# EUROPEAN PATENT SPECIFICATION

(11) **EP 3 858 314 B1**
(45) Date of publication and mention of the grant of the patent: **04.09.2024**
(21) Application number: 19877916.7
(22) Date of filing: 02.08.2019
(51) Int. Cl.: A61F 13/496, A61F 13/15

(54) **ABSORBENT ARTICLE**
ABSORBIERENDER ARTIKEL
ARTICLE ABSORBANT

(30) Priority: 31.10.2018 JP 2018205510
(43) Date of publication of application: 04.08.2021
(73) Proprietor: Unicharm Corporation, Shikokuchuo-shi, Ehime 799-0111 (JP)
(72) Inventor: ICHIKAWA, Makoto, Kanonji-shi, Kagawa 769-1602 (JP); TANAKA, Yoshinori, Kanonji-shi, Kagawa 769-1602 (JP); KAWABATA, Kuniyoshi, Kanonji-shi, Kagawa 769-1602 (JP); MATSUDA, Yoshiyuki, Kanonji-shi, Kagawa 769-1602 (JP)
(74) Representative: Dolleymores
(86) International application number: PCT/JP2019/030591
(87) International publication number: WO 2020/090177

(56) References cited:
- WO-A1-2004/047699
- WO-A1-2010/055767
- WO-A1-2010/055767
- JP-A- 2001 046 432
- JP-A- 2003 024 381
- JP-A- H10 137 289

## Description

### FIELD

The present invention relates to an absorbent article.

### BACKGROUND

There has been know an absorbent article (for example, a pants-type disposable diaper) including a stomach-side portion and a back-side portion and having a joining portion in which at least one end portion of the stomach-side portion and at least one end portion of the back-side portion, both the end portions being in the lateral direction, are joined to each other along the longitudinal direction in a state of being overlapped in the thickness direction. For example, Patent Literature 1 discloses a pants-type absorbent article. In this absorbent article, the side seal portion (joining portion) is divided into an upper region, an intermediate region, and a lower region from the waist opening to the leg opening, the average peeling strength in the longitudinal direction is substantially the same in the upper region and the lower region and is lower in the intermediate region than in other regions. According to Patent Literature 1, it is said that it is possible to achieve both of a seal strength at which a seal can withstand use as a side part and a seal strength at which a seal can be relatively easily broken by this configuration.

### [CITATION LIST]

### [PATENT LITERATURE]

Patent Literature 1: Japanese Unexamined Patent Application Publication No. 2018-477 WO2010/055767 A1 discloses an underpants-type disposable diaper having a pair of side-seal parts formed by the fusing of overlap parts where two side edges of a stomach part and two side edges of a back part are overlapped with each other.

### SUMMARY

### [TECHNICAL PROBLEM]

The joining portion of the absorbent article of Patent Literature 1 as described above includes a plurality of fused portions in which a sheet member on the stomach-side portion and a sheet member on the back-side portion are joined by thermal fusion bonding. Here, the sheet member on the stomach-side portion (hereinafter, also referred to as "stomach-side sheet member") and the sheet member on the back-side portion (hereinafter, also referred to as "back-side sheet member") are usually formed of a nonwoven fabric. However, there may be some unevenness in the thickness of the nonwoven fabric. In addition, when an absorbent article is manufactured, it is usual to perform thermal fusion bonding while continuously transporting a semi-finished product formed of a nonwoven fabric, but there may be a fluctuation (manufacturing error) in the position of the semi-finished product while being transported and the like. Therefore, fused portions in each of which the thermal fusion bonding is not perfect, that is, very heterogeneous fused portions, are formed in a part of the plurality of fused portions in which the stomach-side sheet member and the back-side sheet member are joined by thermal fusion bonding. Incidentally, in the case where the used absorbent article is removed from the wearer, a method of pulling apart the stomach-side sheet member and the back-side sheet member from each other at the joining portions is usually used. That is, the stomach-side sheet member and the back-side sheet member are continuously pulled apart from each other in the longitudinal direction by breaking the sheet members at each fused portion or peripheral portion thereof. However, as described above, in the case where the very heterogeneous fused portions are present in a part of the plurality of fused portions, there is a concern that the following problems may occur when the joining portion is pulled apart. For example, since the peeling strength when the stomach-side sheet member and the back-side sheet member are pulled apart from each other is significantly different in a part of the plurality of fused portions, the sheet members cannot be pulled apart from each other with almost the same force from the starting to the end of the pulling and it is difficult to continuously pull apart the sheet members. Alternatively, during the pulling, tearing occurs in a part of the fused portions and proceeds to a section other than the joining portion of the stomach-side sheet member and the back-side sheet member, for example, the central part where an absorbent body is positioned, and thus it is difficult to pull apart the sheet members to the end at the joining portion or it is necessary to repeat the pulling many times. In the absorbent article of Patent Literature 1, the pattern of the fused portion of the side seal portion (joining portion) is changed depending on the position of the fused portion, but there is a possibility that very heterogeneous fused portions as described above may be formed as well. Therefore, there is a concern that the above problems may occur as well.

Therefore, an object of the present invention is to provide an absorbent article having a joining portion capable of, when removing the absorbent article, continuously and stably separating a stomach-side sheet portion and a back-side sheet portion with almost the same force without breaking the sheet member in a section other than the joining portion.

### [SOLUTION TO PROBLEM]

An absorbent article according to the present invention is (1) an absorbent article having a longitudinal direction, a lateral direction, and a thickness direction that are orthogonal to each other, and including a stomach-side portion including a stomach-side sheet member, a back-side portion including a back-side sheet member, and a joining portion in which at least one end portion of the stomach-side portion and at least one end portion of the back-side portion in the lateral direction are joined to each other in a state of being overlapped in the thickness direction along the longitudinal direction, wherein the joining portion includes a plurality of fused portions which are separated from each other and disposed along the longitudinal direction, each of the plurality of fused portions includes a core section in which the stomach-side sheet member and the back-side sheet member are fused to each other in the thickness direction, and a peripheral wall portion in which the stomach-side sheet member and the back-side sheet member are fused to each other in the thickness direction to extend in a tubular shape in the thickness direction from a peripheral edge of the core portion, a surface of the core portion is flat, in a peeling test of the joining portion in which one end portion of a sample including the joining portion in the longitudinal direction is held by two jigs, and while peeling off the joining portion to widen a distance between the two jigs, a load to be applied between the two jigs is measured, a graph showing the load with respect to the distance has a plurality of protruding portions in which the load temporarily shows local maximum values at respective positions of the plurality of fused portions, and in the peeling test, when the one end portion of the sample in the longitudinal direction is an end portion of either a waist opening side or a leg opening side of the absorbent article, a standard deviation with respect to an average value of the local maximum values in the plurality of protruding portions corresponding to the plurality of fused portions is 3 N or less.

In this absorbent article, the surface of the core portion is flat. Therefore, when each fused portion is viewed, it can be said that the fusion is properly performed and the thickness of the core portion is almost even, and therefore it can be seen that the individual fused portions are substantially homogeneous. Further, since the standard deviation from the average value of the local maximum values of the loads in the plurality of protruding portions corresponding to the plurality of fused portions is 3 N or less in the peeling test of the joining portion, the local maximum value of the load required for peeling each of the plurality of fused portions, that is, the peeling strength of each of the plurality of fused portions is almost the same. Therefore, when the plurality of fused portions are viewed as a whole, it can be said that the fusion is properly performed and the plurality of fused portions are substantially homogeneous as a whole. As described above, since the individual fused portions are substantially homogeneous and the plurality of fused portions are substantially homogeneous as a whole, during the pulling of the joining portion, the occurrence of tearing in a part of the fused portions can be suppressed and the joining portion can be continuously pulled apart with almost the same force from the start to the end of the pulling. The upper limit of the standard deviation of 3 N is set based on the grip strength and grab strength of an elderly person in a manner such that even in the case where the peeling strength between the fused portions is changed when the joining portion is continuously pulled apart, it is possible for the wearer or the caregiver who is an elderly person to cope with the change. Therefore, when removing the absorbent article having the joining portion, it is possible to appropriately separate the stomach-side portion and the back-side portion continuously and stably with almost the same force without breaking the sheet member in a section other than the joining portion. The details of the flatness of the surface of the core portion and the peeling test of the joining portion will be described later.

The absorbent article of the present invention may be (2) the absorbent article according to (1) above, wherein in the peeling test, the average value of the local maximum values in the plurality of protruding portions corresponding to the plurality of fused portions is 2 N or more and 12 N or less. In the absorbent article, the average value of the local maximum values of the load (peeling strength) in the peeling test of the joining portion is 2 N or more and 12 N or less. Therefore, each fused portion can be pulled apart from the stomach-side sheet member or the back-side sheet member with a relatively small force, whereby the joining portion can be pulled apart with a relatively small force. That is, it is possible to separate the stomach-side portion and the back-side portion continuously and stably with almost the same force and easily with a relatively small force without breaking the sheet member in a section excluding the joining portion. The upper limit of the peeling strength of 12 N is set based on the grip strength and grab strength of an elderly person in a manner such that even in the case where the peeling strength in the fused portion is high when the joining portion is continuously pulled apart, it is possible for the wearer or the caregiver who is an elderly person to peel off the fused portion.

The absorbent article of the present invention may be (3) the absorbent article according to (1) or (2) above, wherein in the peeling test, an energy required for peeling off the joining portion is 2 J or more and 7 J or less. In the absorbent article, since the energy required for peeling off the joining portion in the peeling test of the joining portion is 2 J or more and 7 J or less, it is possible to pull apart the joining portion with a relatively small amount of work. That is, it is possible to separate the stomach-side portion and the back-side portion continuously and stably with almost the same force and easily with a relatively small amount of work without breaking the sheet member in a section other than the joining portion. The upper limit of the energy of 7 J required to peel off the joining portion is set based on the grip strength and grab strength of an elderly person in a manner such that even in the case where the energy is high when the joining portion is continuously pulled apart, it is possible for the wearer or the caregiver who is an elderly person to continuously peel off the fused portion.

The absorbent article of the present invention is (4) the absorbent article according to any one of (1) to (3) above, where in a puncture test of the stomach-side sheet member and the back-side sheet member, a maximum load of the stomach-side sheet member and a maximum load of the back-side sheet member are 10 N or more and 25 N or less. In the absorbent article, since the maximum load (breaking strength) of the stomach-side sheet member and the maximum load (breaking strength) of the back-side sheet member are 10 N or more and 25 N or less, it is possible to easily break the sheet members with a relatively small force when the stomach-side portion and the back-side portion are separated. Due to that, it is possible to separate the stomach-side portion and the back-side portion continuously and stably with almost the same force and easily with a relatively small force without breaking the sheet member in a section other than the joining portion. The upper limit of the breaking strength of 25 N of the sheet member is set based on the grip strength and grab strength of an elderly person in a manner such that even in the case where the breaking strength of the sheet member is high when the joining portion is continuously pulled apart, it is possible for the wearer or the caregiver who is an elderly person to break the sheet member in the fused portion.

The absorbent article of the present invention is (5) the absorbent article according to (4) above, wherein in the puncture test, a difference between the maximum load of the stomach-side sheet member and the maximum load of the back-side sheet member is 1 N or more and 10 N or less. In the absorbent article, since the difference between the maximum load (breaking strength) of the stomach-side sheet member and the maximum load (breaking strength) of the back-side sheet member in the puncture test is 1 N or more and 10 N or less, it is possible to relatively easily break a sheet member having a relatively small maximum load from each fused portion when the stomach-side portion and the back-side portion are separated. Due to that, it is possible to separate the stomach-side portion and the back-side portion continuously and stably with almost the same force and more easily with a relatively small force without breaking the sheet member in a section excluding the joining portion. The upper limit of the difference in breaking strength of 10 N is set based on the grip strength and grab strength of an elderly person in a manner such that even in the case where the difference in the breaking strength of the sheet member between fused portions is changed when the joining portion is continuously pulled apart, it is possible for the wearer or the caregiver who is an elderly person to cope with the change.

The peeling test may be performed by using the one end portion of the sample in the longitudinal direction as each of an end portion on the waist opening side and an end portion on the leg opening side. In this absorbent article, each of the end portion of the waist opening side and the end portion of the leg opening side is held by two jigs and subjected to the peeling test. Therefore, in both case, the standard deviation of the local maximum values at least in the protruding portions is 3 N or less and the like. Therefore, when the absorbent article having the joining portion is removed, it is possible to appropriately separate the stomach-side portion and the back-side portion continuously and stably with almost the same force without breaking the sheet member in a section other than the joining portion regardless of the positions where the pulling is started (the end portion on the waist opening side and the end portion on the leg opening side).

### [ADVANTAGEOUS EFFECT OF THE INVENTION]

According to the present invention, it is possible to provide an absorbent article having a joining portion capable of, when removing the absorbent article, continuously and stably separating a stomach-side sheet portion and a back-side sheet portion with almost the same force without breaking a sheet member in a section other than the joining portion.

### BRIEF DESCRIPTION OF DRAWINGS

Fig. 1 is a perspective view showing a constitution example of a disposable diaper according to an embodiment.
Fig. 2 is a plan view showing an unfolded state of the disposable diaper.
Fig. 3 is a schematic view of a stomach-side portion and a back-side portion in a joining portion as seen from a side of an end portion in a lateral direction.
Fig. 4 is a schematic view for describing a method of forming a joining portion in a manufacturing step.
Fig. 5 is a plan view showing a constitution example of the joining portion in the disposable diaper.
Fig. 6 is a plan view for describing a constitution example of a plurality of fused portions in the joining portion.
Fig. 7 is a plan view and a perspective view showing a constitution example of the fused portion.
Fig. 8 is a schematic view for describing a line roughness test of the fused portion.
Fig. 9 is a schematic view for describing a peeling test of the joining portion.
Fig. 10 is a graph showing a relationship between a load and a distance in the peeling test.
Fig. 11 is a schematic view for describing an appearance of the fused portion being pulled apart.
Fig. 12 is a schematic view for describing a puncture test of a sheet member.
Fig. 13 is a plan view for describing another constitution example of the plurality of fused portions in the joining portion.
Fig. 14 is a plan view showing another constitution example of the fused portion.

### DESCRIPTION OF EMBODIMENTS

Hereinafter, an absorbent article according to an embodiment of the present invention will be described using a pants-type disposable diaper (hereinafter, also simply referred to as a "disposable diaper") as an example. However, the present invention is not limited to the example and is applicable to a variety of absorbent articles without departing from the subject matter of the present invention. Examples of the absorbent article include so-called three-piece type and two-piece type disposable diapers.

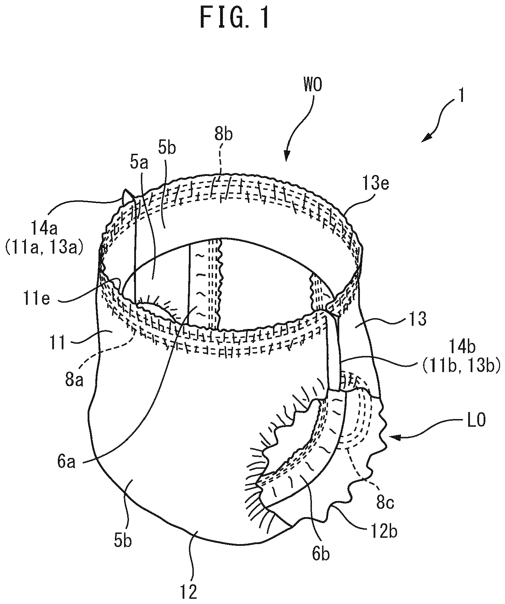

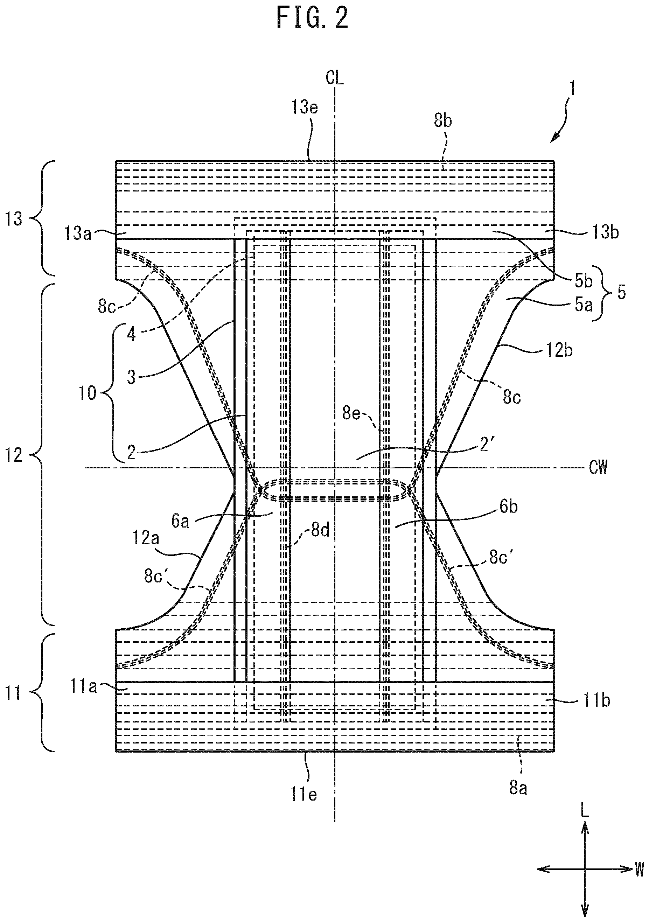

Fig. 1 and Fig. 2 are views showing a disposable diaper 1 according to the present embodiment. Fig. 1 is a perspective view showing a constitution example of the disposable diaper 1, and Fig. 2 is a plan view showing a state in which the disposable diaper 1 is unfolded. In the state shown in Fig. 2, the disposable diaper 1 has a longitudinal direction L, a lateral direction W, and a thickness direction T that are orthogonal to each other and has a central axis CL that passes through the center in the lateral direction W and extends in the longitudinal direction L and a central axis CW that passes through the center in the longitudinal direction L and extends in the lateral direction W. Here, the orientation toward and the side facing the central axis CL are respectively defined as inward and the inner side in the lateral direction W, and the orientation and the side away from the central axis CL are respectively defined as outward and the outer side in the lateral direction W. On the other hand, the orientation toward and the side facing the central axis CW are respectively defined as inward and the inner side in the longitudinal direction L, and the orientation and the side away from the central axis CW are respectively defined as outward and the outer side in the longitudinal direction L. Further, viewing the disposable diaper 1 placed on a plane in the thickness direction T from the vertically upper side of the plane is referred to as "plan view", the shape grasped in the plan view is referred to as "planar shape", and any direction in the plane including the longitudinal direction L and the lateral direction W is referred to as "in-plane direction". The terms "skin side" and "non-skin side" respectively mean sides that are relatively close to and far from the skin surface of a wearer in the thickness direction T of the disposable diaper 1 when the wearer wears the disposable diaper 1. It should be noted that the respective definitions described above will also be used for each material for the disposable diaper 1 or the disposable diaper 1 in the state shown in Fig. 1.

The disposable diaper 1 includes a stomach-side portion 11, a back-side portion 13, and an intermediate portion 12 between the stomach-side portion 11 and the back-side portion 13. The stomach-side portion 11 is a portion of the disposable diaper 1 that faces the abdomen of a wearer. The intermediate portion 12 is a portion of the disposable diaper 1 that faces the crotch portion of the wearer. The back-side portion 13 is a portion of the disposable diaper 1 that faces the hip portion or back portion of the wearer. The stomach-side portion 11 and the back-side portion 13 are joined to each other with a pair of joining portions 14a and 14b positioned at both ends of the stomach-side portion 11 and the back-side portion 13 in the lateral direction W. The joining portion 14a is formed of one end portion 11a of the stomach-side portion 11 in the lateral direction W and one end portion 13a of the back-side portion 13 in the lateral direction W that are joined to each other along the longitudinal direction L in a state of being overlapped in the thickness direction T. Similarly, the joining portion 14b is formed of the other end portion 11b of the stomach-side portion 11 in the lateral direction W and the other end portion 13b of the back-side portion 13 in the lateral direction W that are joined to each other along the longitudinal direction L in a state of being overlapped in the thickness direction T. In this case, in the disposable diaper 1, a waist opening WO through which the waist of a wearer passes is defined by an end portion 11e of the stomach-side portion 11 on the outer side in the longitudinal direction L and an end portion 13e of the back-side portion 13 on the outer side in the longitudinal direction L. In addition, in the disposable diaper 1, a pair of leg openings LO and LO through which the legs of the wearer pass are defined by side portions 12a and 12b of the intermediate portion 12 on both sides in the lateral direction W. It should be noted that the longitudinal direction, the lateral direction, and the thickness direction of each joining portion 14a or 14b in the state shown in Fig. 1 are the same as the longitudinal direction L, the lateral direction W, and the thickness direction T in the state shown in Fig. 2.

In the present embodiment, in the unfolded state shown in Fig. 2, the stomach-side portion 11 and the back-side portion 13 each have an almost rectangular shape that extends in the lateral direction W and are separated from each other in the longitudinal direction L. The intermediate portion 12 is positioned between the stomach-side portion 11 and the back-side portion 13, and both side edges in the lateral direction W are recessed inward in the lateral direction W. The stomach-side portion 11, the intermediate portion 12, and the back-side portion 13 are integrally formed with each other. In another embodiment (not shown), the stomach-side portion 11, the intermediate portion 12, and the back-side portion 13 are formed separately from each other.

In the present embodiment, the disposable diaper 1 is provided with a plurality of elastic members 8a and 8b for waist gather on the stomach-side portion 11 and the back-side portion 13, respectively. The plurality of elastic members 8a extend along the end portion 11e of the stomach-side portion 11 in the longitudinal direction L from one end portion 11a to the other end portion 11b in the lateral direction W and are disposed at intervals in the longitudinal direction L. Similarly, the plurality of elastic members 8b extend along the end portion 13e of the back-side portion 13 in the longitudinal direction L from one end portion 13a to the other end portion 13b in the lateral direction W and are disposed at intervals in the longitudinal direction L. The plurality of elastic members 8a and 8b stretch and contract the waist opening WO and are exemplified by elastic strings.

In the present embodiment, the disposable diaper 1 includes a plurality of elastic member 8c and 8c' for leg gathers from the intermediate portion 12 to the back-side portion 13 and the stomach-side portion 11. The plurality of elastic members 8c and 8c' are continuously disposed at intervals to extend along the lateral direction W in the center of the intermediate portion 12 in the longitudinal direction L, reach both end portions of the intermediate portion 12 in the lateral direction W, and then extend along the longitudinal direction L up to the end portions 13a and 13b of the back-side portion 13 and the end portions 11a and 11b of the stomach-side portion 11, respectively. The plurality of elastic members 8c and 8c' stretch and contract the pair of leg openings LO and LO respectively and are exemplified by elastic strings.

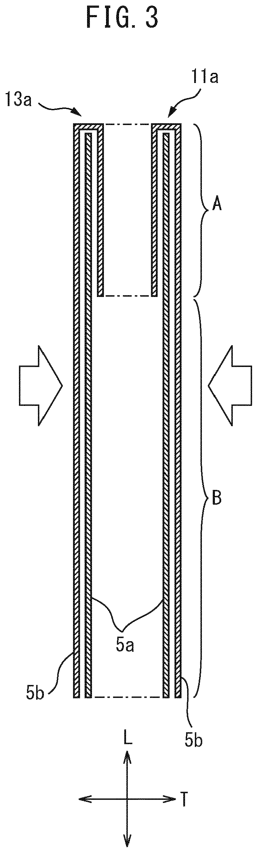

In the present embodiment, the stomach-side portion 11, the intermediate portion 12, and the back-side portion 13 of the disposable diaper 1 are formed of a liquid-impermeable cover sheet (sheet member) 5. Fig. 3 is a schematic view of the stomach-side portion 11 and the back-side portion 13 in the joining portion 14a or 14b as seen from the side of the end portion in the lateral direction W. However, for the sake of clarity, each cover sheet 5 is shown separately, and the description of the elastic member is omitted. The cover sheet 5 includes a cover sheet 5a that is positioned on the skin side and a cover sheet 5b that is positioned on the non-skin side, and the cover sheet 5a and the cover sheet 5b are laminated together in the thickness direction T and joined to each other with an adhesive or the like. Both end portions of the cover sheet 5b in the longitudinal direction L are folded back toward the skin side to cover both end portions of the cover sheet 5a in the longitudinal direction L. In this case, the cover sheet 5b in the folded-back positions in the stomach-side portion 11 and the back-side portion 13 constitutes the end portion 11e of the stomach-side portion 11 and the end portion 13e of the back-side portion 13, respectively. A region A is a region where three layers of the cover sheet are present, and a region B is a region where two layers of the cover sheet are present. Therefore, the end portion 11e of the stomach-side portion 11 and the end portion 13e of the back-side portion 13 have a three-layer structure in which three cover sheets 5b, 5a, and 5b are laminated together. The length of each three-layer structure in the longitudinal direction L is, for example, 10 mm to 100 mm. In the present embodiment, the plurality of elastic members 8a, 8b, 8c, and 8c' are positioned between the cover sheet 5a and the cover sheet 5b.

Examples of the cover sheet 5 include any liquid-impermeable sheets such as liquid-impermeable nonwoven fabric or synthetic resin films, composite sheets thereof, SB nonwoven fabric, and SMS nonwoven fabric. Examples of the material of the cover sheet 5 include polyolefin-based materials such as polypropylene or polyethylene. The basis weight of the cover sheet 5 is, for example, 5 to 100 g/m² and preferably 10 to 50 g/m². The dimension (thickness) of the cover sheet 5 in the thickness direction T is, for example, 0.1 to 5 mm and preferably 0.1 to 2 mm. In another embodiment, the cover sheet 5 includes one cover sheet or two or more cover sheets (not shown). In still another embodiment, the cover sheet 5b may not be folded back.

In the present embodiment, the disposable diaper 1 includes an absorbent main body 10 on a skin side surface of the cover sheet 5. The absorbent main body 10 has a substantially rectangular shape and includes a liquid-permeable top-surface sheet 2, a liquid-impermeable back-surface sheet 3, and an absorbent body 4 that is positioned between the top-surface sheet 2 and the back-surface sheet 3 and absorbs and holds liquid. Examples of the top-surface sheet 2 include liquid-permeable nonwoven fabric or woven fabric, synthetic resin films having a liquid permeable hole formed therein, composite sheet thereof, and the like. Examples of the back-surface sheet 3 include liquid-impermeable nonwoven fabric or synthetic resin films, composite sheets thereof, SMS nonwoven fabric, and the like. In the present embodiment, the absorbent body 4 includes an absorbent body core that absorbs and holds liquid and a core wrap that contains the absorbent body core. As the absorbent body 4, pulp fibers, synthetic fibers, absorbent polymers, or the like is exemplified. The absorbent body 4 is respectively joined to the top-surface sheet 2 and the back-surface sheet 3 with an adhesive or the like, and the top-surface sheet 2 and the back-surface sheet 3 are joined to each other with the adhesive at the peripheral edge parts. The adhesive is a well-known material that is common for the disposable diaper 1, and examples thereof include thermoplastic adhesives. The shape of the absorbent main body 10 is not limited to the above example as long as the shape is long in the longitudinal direction L, and examples thereof include a rectangular shape with rounded corners, a rectangular shape whose short sides protrude to the outer side and curved, and an hourglass shape.

In the present embodiment, the disposable diaper 1 includes liquid-impermeable leakproof walls 6a and 6b. The leakproof walls 6a and 6b are disposed along the longitudinal direction L on both sides of the top-surface sheet 2 in the lateral direction W. In the end portions of the leakproof walls 6a and 6b on the inner side in the lateral direction W, a plurality of elastic members 8d and 8e that respectively extend in the longitudinal direction L and are aligned at intervals in the lateral direction W are disposed. The plurality of elastic members 8d and 8e respectively stretch and contract the leakproof walls 6a and 6b. As the elastic members 8d and 8e, elastic strings are exemplified.

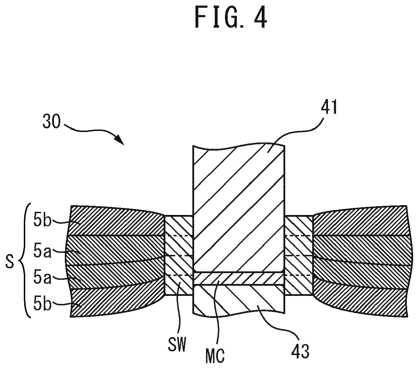

Next, an example of a method for manufacturing the disposable diaper 1 will be described. First, a disposable diaper continuous body (not shown) is formed in which disposable diapers in the unfolded state shown in Fig. 2 are continuously coupled to each other in the lateral direction W. The disposable diaper continuous body has a structure in which the end portion 11b of the stomach-side portion 11 and the end portion 13b of the back-side portion 13 of one of disposable diapers adjacent to each other are coupled to the end portion 11a of the stomach-side portion 11 and the end portion 13a of the back-side portion 13 of the other disposable diaper. The disposable diaper continuous body is transported in a manner such that the lateral direction W becomes parallel to a machine direction MD. Subsequently, the disposable diaper continuous body is transported in the machine direction MD and folded in the longitudinal direction L along the central axis CW with a folding device (not shown). That is, in the disposable diaper continuous body, the end portions 11a of the stomach-side portions 11 and the end portions 13a of the back-side portions 13 of the respective disposable diapers are laminated together, and the end portions 11b of the stomach-side portions 11 and the end portions 13b of the back-side portions 13 are laminated together. Next, in the disposable diaper continuous body, both end portions of the stomach-side portions 11 and the back-side portions 13 are respectively joined to each other by ultrasonic wave sealing using a joining portion forming apparatus (not shown). Fig. 4 is a schematic view for describing a method of forming the joining portion. This view shows a part of the region B in Fig. 3. Specifically, a laminated portion of the end portion 11a of the stomach-side portion 11 and the end portion 13a of the back-side portion 13 in a disposable diaper continuous body S, and a laminated portion of the end portion 11b of the stomach-side portion 11 and the end portion 13b of the back-side portion 13 are respectively pinched between an ultrasonic horn 43 of a joining portion forming apparatus and a protruding portion 41 of an anvil. Energy of a predetermined ultrasonic wave is supplied to the pinched portions for a predetermined period of time. Therefore, the plurality of cover sheet 5b, 5a, 5a, and 5b in the laminated portions are fused to each other, thereby forming fused portions 30 each including a core portion MC and a peripheral wall portion SW. Both end portions of the stomach-side portion 11 and the back-side portion 13 are joined to each other with the fused portions 30, and the pair of joining portions 14a and 14b is formed. The shape of the core portion MC and the peripheral wall portion SW can be controlled by the magnitude of the energy supplied from the joining portion forming apparatus, the timing and period of supply of the energy, and the like. Examples of such joining portion forming apparatus include the apparatuses described in Japanese Unexamined Patent Application Publication No. 2004-298413 and Japanese Unexamined Patent Application Publication No. 2006-192902. For example, the following control is possible: the transport speed is set to be slower than usual, energy is set to be higher than usual, or the size of the protruding portion 41 is set to be smaller than usual, thereby increasing the density per unit area of energy that is to be supplied to a region in which the fused portion of the cover sheets is to be formed and efficiently using energy, which enables energy to be sufficiently given not only to the front end of the protruding portion 41 but also to the periphery of the protruding portion 41. Therefore, it is possible to form not only the core portion MC, which is usually formed at the front end of the protruding portion 41, but also the peripheral wall portion SW in the periphery of the protruding portion 41. It should be noted that the protruding portion 41 may be formed in the ultrasonic horn and the anvil may be formed flat.

Next, the constitution of the joining portions 14a and 14b will be described. Here, the joining portion 14a and the joining portion 14b are almost the same in terms of the constitution except for the difference in position (right and left) in the lateral direction W. Therefore, hereinafter, the constitution of the joining portion 14a will be mainly described, and the constitution of the joining portion 14b will not be described.

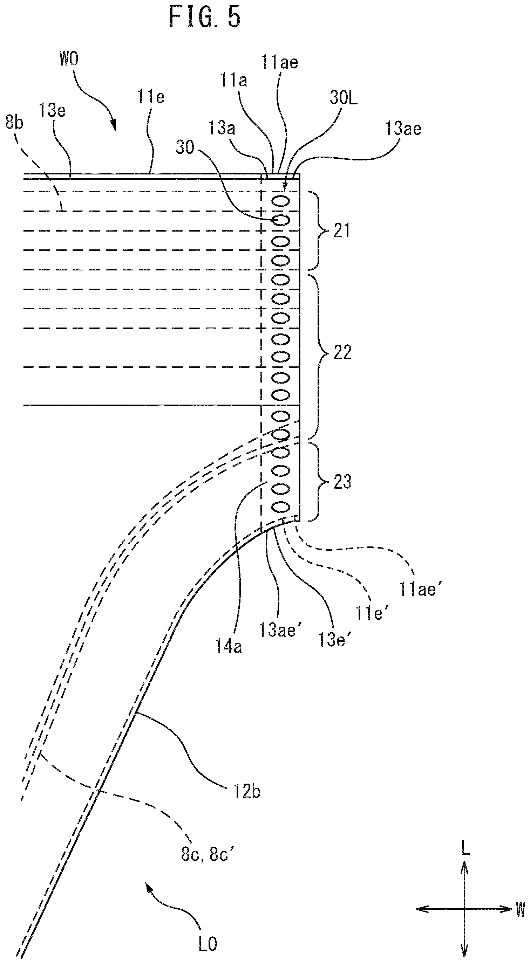

Fig. 5 is a plan view schematically showing a constitution example of the joining portion 14a in the disposable diaper 1. Fig. 5 is a view of the joining portion 14a as seen from the back-side portion 13 side. In the present embodiment, in the joining portion 14a, an end edge 11ae of the end portion 11a in the stomach-side portion 11 on the outer side in the longitudinal direction L and an end edge 13ae of the end portion 13a in the back-side portion 13 on the outer side in the longitudinal direction L are almost overlapped with each other. The dimensions of the joining portion 14a in the longitudinal direction L and the lateral direction W are, for example, 50 to 250 mm and 3 to 20 mm, respectively. In another embodiment, the end edge 11ae and the end edge 13ae deviate from each other in the longitudinal direction L by a predetermined length (for example, 0.5 to 10 mm) (not shown).

The joining portion 14a includes a plurality of the fused portions 30 that are separated from each other and disposed along the longitudinal direction L. In this case, the end portion 11a and the end portion 13a are joined to each other with at least the plurality of fused portions 30. The stomach-side portion and the back-side portion are not joined to each other at portions in the joining portion 14a at which the fused portions 30 are not formed. In another embodiment, portions in the joining portion 14a at which the fused portions 30 are not formed are fully or partially joined to each other with an adhesive (for example, hot-melt adhesive) (not shown).

In the present embodiment, in the joining portion 14a, the plurality of fused portions 30 are disposed to be aligned side by side at intervals along the longitudinal direction L, thereby constituting a fused portion line 30L. The fused portion line 30L is one line, and the intervals between the fused portions 30 in the longitudinal direction L are constant. However, in another embodiment, a plurality of the fused portion lines 30L are aligned side by side at intervals in the lateral direction W (not shown). In addition, in still another embodiment, in all or part of the fused portion line 30L, the intervals between the fused portions 30 in the longitudinal direction L vary depending on positions (not shown).

In the present embodiment, the plurality of elastic members 8a are provided between the cover sheets 5a and 5b in the stomach-side portion 11 to extend in the lateral direction W up to the joining portions 14a and 14b and to be aligned at intervals in the longitudinal direction L. Similarly, the plurality of elastic members 8b are provided between the cover sheets 5a and 5b in the back-side portion 13 to extend in the lateral direction W up to the joining portions 14a and 14b and to be aligned at intervals in the longitudinal direction L. In addition, the plurality of elastic members 8a and 8b (preferably also the elastic members 8c and 8c') are disposed not to overlap with the plurality of fused portions 30 in the thickness directions T at least in the joining portion 14a.

In the present embodiment, the joining portion 14a has, in the longitudinal direction L, a waist-side end region 21 including the end portion on the waist opening WO side, a leg-side end region 23 including the end portion on the leg opening LO side, and a central region 22 between both regions. The waist-side end region 21 and the leg-side end region 23 are regions that range from 1/8 to 1/3 of the joining portion 14a in the longitudinal direction L from the end edges of the waist opening WO and the leg opening LO, respectively, and the central region 22 is the remaining region. The waist-side end region 21 and the leg-side end region 23 have the same length in the longitudinal direction L. In each region, the fused portions 30 are disposed in the same number of lines at the same interval (with a manufacturing error). In another embodiment, the waist-side end region 21 and the leg-side end region 23 have different lengths in the longitudinal direction L (not shown). In another embodiment, in at least one region, at least one of the size, interval, and number of lines of the fused portions 30 is different from that of other regions (not shown).

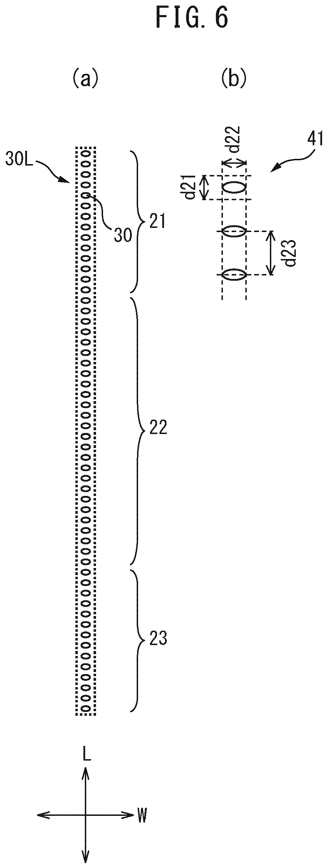

Fig. 6 is a plan view for describing a constitution example of the plurality of fused portions 30 in the joining portion 14a. Fig. 6(a) shows the fused portions 30 (fused portion line 30L) as seen from the side of the back-side portion 13 in the thickness direction T, and Fig. 6(b) shows the shape and disposition of the protruding portion 41 at the front end portion of the anvil (or ultrasonic horn) in which the fused portion 30 is formed by the ultrasonic wave sealing method. In the present embodiment, as the fused portion line 30L, the plurality of fused portions 30 in the waist-side end region 21, the central region 22, and the leg-side end region 23 all have the same planar shape. In other words, in the case of forming the respective fused portions 30 in the respective regions by the ultrasonic wave sealing method, the planar shapes of the protruding portions 41 of the anvil for the respective fused portions 30 in the respective regions are the same. Here, the planar shape of the protruding portion 41 is not particularly limited, and examples thereof include a circular shape, an elliptical shape, a rectangular shape, a rounded rectangular shape, a polygonal shape, and the like. In the present embodiment, the planar shape of the protruding portion 41 is an elliptical shape, a dimension d21 (minor axis) in the longitudinal direction L is smaller than a dimension d22 (major axis) in the lateral direction W, and the sizes thereof are, for example, 0.5 mm to 5 mm. A distance d23 between the protruding portions 41 adjacent to each other in the longitudinal direction L (the distance between the major axes) is, for example, 1 mm to 30 mm.

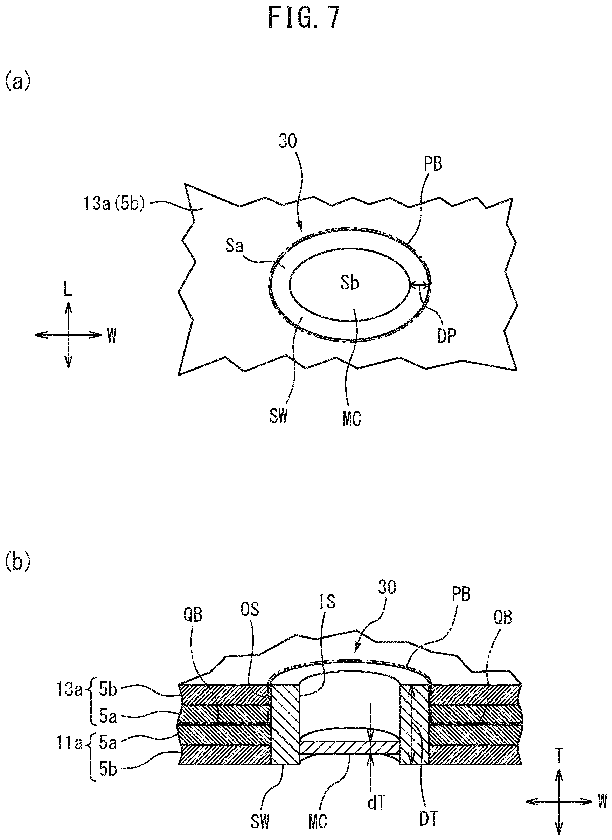

Next, the constitution of the fused portion 30 will be further described. Here, the constitution of the fused portion 30 in the central region 22 will be described. It should be noted that the constitution of the fused portion 30 in the leg-side end region 23 and the constitution of the waist-side end region 21 (excluding the number of the cover sheets 5) are also the same as the constitution of the fused portion 30 in the central region 22 and thus will not be described again. Fig. 7 is a plan view and a perspective view showing an example of the constitution of the fused portion 30. Here, Fig. 7(a) is a plan view of the fused portion 30 in the central region 22, and Fig. 7(b) is a perspective view including a cross section of the fused portion 30 in the central region 22 as seen in the longitudinal direction L. The fused portion 30 includes the core portion MC and the peripheral wall portion SW.

In the present embodiment, the core portion MC has a constitution in which the plurality of cover sheets 5a and 5b in the stomach-side portion 11 and the plurality of cover sheets 5a and 5b in the back-side portion 13 are fused to each other in the thickness direction T. The core portion MC is formed through a process that the plurality of cover sheets 5b, 5a, 5a, and 5b are pinched between the front end portion of the ultrasonic horn and the protruding portion of the anvil, melted by energy that is supplied from the ultrasonic horn, while compressed in the thickness direction T, and then solidified. Therefore, the core portion MC is joined extremely strongly in the thickness direction T. A dimension (thickness) dT of the core portion MC in the thickness direction T is, for example, 20 to 500 µm. The dimension dT of the core portion MC is significantly smaller than the dimension of the plurality of cover sheets 5b, 5a, 5a, and 5b in the stomach-side portion 11 and the back-side portion 13 that are simply laminated together in the thickness direction T (for example, 200 to 5000 µm). When dT is smaller than 20 µm, the cover sheets are not stably fused to each other, and a hole is easily opened during fusion. When dT is more than 500 µm, the cover sheets are not fully fused to each other, and there is a concern that the cover sheets may be easily broken.

In addition, in the present embodiment, the planar shape of the core portion MC is almost the same as the planar shape of the protruding portion 41 of the anvil, and examples thereof include a circular shape, an elliptical shape, a rectangular shape, a rounded rectangular shape, a polygonal shape, and the like. In the present embodiment, the planar shape of the core portion is an elliptical shape. At this time, the dimension in the longitudinal direction L and the dimension in the lateral direction W are almost the same as the dimension d21 in the longitudinal direction L and the dimension d22 in the lateral direction W of the protruding portion 41 and are 0.5 mm to 5 mm. In the case where each dimension of the core portion MC is smaller than 0.5 mm, the fused portions 30 are too small and are easily broken, and there is a concern that the joining portion 14a may be unintentionally peeled off while the disposable diaper 1 is worn. In the case where each dimension of the core portion MC is more than 5 mm, the fused portion 30 is too large, and there is a concern that a wearer may sense discomfort by feeling hardness. In addition, the distance between the core portions MC adjacent to each other is almost the same as the distance between the protruding portions 41 adjacent to each other and is 1 mm to 30 mm. In the case where the distance is smaller than 1 mm, the number of the fused portions 30 in the joining portion 14a increases, and there is a concern that the disposable diaper 1 may be less likely to be pulled apart when attempted to be removed. In the case where the distance is more than 30 mm, the number of the fused portions 30 decreases, and there is a concern that the joining portion 14a may be unintentionally peeled off while the disposable diaper 1 is worn.

Furthermore, in the present embodiment, in the fused portion 30, the position of the core portion MC in the thickness direction T is shifted toward the stomach-side portion 11 or the back-side portion 13 from the boundary between the stomach-side portion 11 and the back-side portion 13. Here, the position of the core portion MC in the thickness direction T is defined as the position of the center of the core portion MC in the thickness direction T. In the case where the core portion MC is not flat, the position of the core portion MC in the thickness direction T is defined as the position of the center of the core portion MC in the thickness direction T in the coupling portion of the core portion MC and the peripheral wall portion SW in the longitudinal direction L. For example, in the example of Fig. 7(b), the position of the core portion MC in the thickness direction T is shifted toward the stomach-side portion 11 side and is a position between the cover sheet 5a and the cover sheet 5b of the stomach-side portion 11. The shift in the thickness direction T from the boundary between the stomach-side portion 11 and the back-side portion 13 is, for example, 10 to 500 µm. In another embodiment, in the thickness direction T, the position of the end surface of the core portion MC coincides with the position of the end surface of the cover sheet 5 in the stomach-side portion 11 or the back-side portion 13 (not shown).

In the present embodiment, the peripheral wall portion SW has a constitution in which the plurality of cover sheets 5a, 5b of the stomach-side portion 11 and the plurality of cover sheets 5a, 5b of the back-side portion 13 are fused to each other in the thickness direction T to extend in a tubular shape in the thickness direction T from the peripheral edge of the core portion MC while surrounding the core portion MC. In the present embodiment, the peripheral wall portion SW is formed through a process that the plurality of cover sheet 5b, 5a, 5a, and 5b are melted by energy that is supplied from the front end portion of the ultrasonic horn or heat that is generated during the fusion of the core portion MC, while slightly compressed in the thickness direction T due to the compression of the core portion MC, and then solidified. Therefore, the peripheral wall portion SW is joined relatively strongly in the thickness direction T. A dimension (thickness) DT of the peripheral wall portion SW in the thickness direction T is, for example, 100 to 1000 µm. The dimension DT of the peripheral wall portion SW is smaller than the dimension of the plurality of cover sheets 5b, 5a, 5a, and 5b in the stomach-side portion 11 and the back-side portion 13 that are simply laminated together in the thickness direction T (for example, 200 to 5000 µm). However, since the peripheral wall portion SW is not pressurized by the front end portion of the ultrasonic horn or the protruding portion 41 of the anvil, the dimension DT of the peripheral wall portion SW is larger than the dimension dT of the core portion MC (for example, 20 to 500 µm). When the size of DT is smaller than 100 µm, the breaking strength of the peripheral wall portion SW becomes weak, and there is a concern that the cover sheet may be broken in an unexpected direction at the time of removing the disposable diaper. When the size of DT is more than 1000 µm, there is a concern that a wearer or a caregiver may sense discomfort when the peripheral wall portion comes into contact with the skin of the wearer or the caregiver.

Here, the peripheral wall portion SW has a tubular form and has an outer surface OS that constitutes the surface of the tube on the outer side and an inner surface IS that constitutes the surface of the tube on the inner side. Therefore, the dimension of the peripheral wall portion SW in the in-plane direction (thickness) can be said as the distance between the outer surface OS and the inner surface IS. When the fused portion 30 is cut in a cross section parallel to the thickness direction T, the outer surface OS and the inner surface IS are the surface on the outer side and the surface on the inner side of the peripheral wall portion provided to stand along the thickness direction T from the peripheral edge of the core portion MC in the cross section. That perpendicular cross section can be obtained by observation with, for example, a scanning electron microscope. The outer surface OS is a boundary surface between a portion of the cover sheet 5 where there is no fused portion 30 and the fused portion 30. Therefore, a fiber in the cover sheet 5 is released from the surface of the outer surface OS, and accordingly, the outer surface OS is a somewhat uneven surface. On the other hand, the inner surface IS is a surface in contact with the protruding portion 41 of the anvil. Therefore, although there is the case where the fiber in the cover sheet 5 remains in a part of the inner surface IS, the inner surface IS is a relatively flat surface. In addition, the outer surface OS and the inner surface IS do not necessarily intersect perpendicularly with the surface of the core portion MC and may be inclined to some extent (for example, ±30° with respect to the vertical direction). Here, the tubular form (or tubular shape) refers to a long and narrow hollow shape or a pipe shape. The tubular form may bend or meander as long as the tubular shape extends in the thickness direction T, may have a variety of shapes such as a circular shape, an elliptical shape, a polygonal shape, and a combined shape on a cross section perpendicular to the thickness direction T, and may have a shape and an area on a cross section perpendicular to the thickness direction T that are not constant in the thickness direction T.

In addition, in the present embodiment, the planar shape of the peripheral wall portion SW has a ring shape that surrounds the core portion MC in an almost even width DP, and examples thereof include a circular ring, an elliptical ring, a rectangular ring, a rounded rectangular ring, a polygonal ring, and the like. In the present embodiment, the planar shape of the peripheral wall portion SW is an elliptical ring. In addition, in the peripheral wall portion SW, the variation of the width DP depending on the position in the ring is small, the width DP rarely becomes partially thin or thick abruptly, and the shape is almost even. That is, the width DP (or wall thickness) of the peripheral wall portion SW in the in-plane direction is almost even. Here, the expression "even" refers to the fact that, in the peripheral wall portion SW, there is no place where the width DP becomes as small a value as equal to or smaller than 1/2 of the average value or there is no place where the width DP becomes as large a value as equal to or larger than twice the average value. It should be noted that, when the width DP is not constant in the thickness direction T, the width at the boundary between the stomach-side portion 11 and the back-side portion 13 is used as the width DP. The size of the width DP is, for example, 50 to 400 µm. In the case where the width DP is smaller than 50 µm, the peripheral wall portion SW is too thin and is easily broken, and there is a concern that the joining portion 14a may unintentionally peel off while the disposable diaper 1 is worn. In the case where the DP is more than 400 µm, the peripheral wall portion SW is too thick, and there is a concern that a wearer may sense discomfort by feeling hardness.

In the present embodiment, in the fused portion 30, an area Sa of the peripheral wall portion SW is in a range of 5% to 60% as seen in the thickness direction T (in a plan view) when an area Sb of the core portion MC is regarded as 100%. The area Sa of the peripheral wall portion SW is preferably in a range of 5% to 55% and more preferably in a range of 5% to 50% of the area Sb of the core portion MC. When the area Sa of the peripheral wall portion SW is less than 5%, the breaking strength of the peripheral wall portion SW decreases, and there is a concern that the peripheral wall portion SW itself may be broken at the time of removing the disposable diaper. When the area Sa of the peripheral wall portion SW is more than 60%, the peripheral wall portion SW becomes too large, and there is a concern that a wearer may sense discomfort by feeling hardness.

Incidentally, in the case where the above-mentioned disposable diaper 1 is removed from the wearer after use, the disposable diaper 1 is removed by peeling off the stomach-side portion 11 and the back-side portion 13 from each other at each of the joining portions 14a and 14b instead of removing the disposable diaper 1 while maintaining the original shape as if the underwear is removed. That is, the cover sheet 5 on the stomach-side portion 11 and the cover sheet 5 on the back-side portion 13 are continuously pulled apart from each other in the longitudinal direction L, and the disposable diaper 1 is removed. In that case, in order to facilitate continuous pulling, as will be described later, it is important that the individual fused portions 30 are substantially homogeneously formed and the plurality of fused portions are substantially homogeneous as a whole.

Whether or not the individual fused portions 30 are substantially homogeneous can be evaluated by the flatness of the surface of the core portion MC. The reason therefor is as follows. In the case where the flatness of the surface of the core portion MC is high, the unevenness of the surface of the core portion MC is small, so that the thickness dT of the core portion MC can be considered to be almost even. Due to that, it can be said that the core portion MC is substantially homogeneous as a whole. Since the core portion MC is substantially homogeneous as a whole, it can be said that the thickness DT and width DP of the peripheral wall portion SW formed at the same time as the core portion MC by ultrasonic wave sealing are also substantially homogeneous. That is, it is possible to evaluate whether or not each fused portion 30 is substantially homogeneous based on the flatness of the surface of the core portion MC.

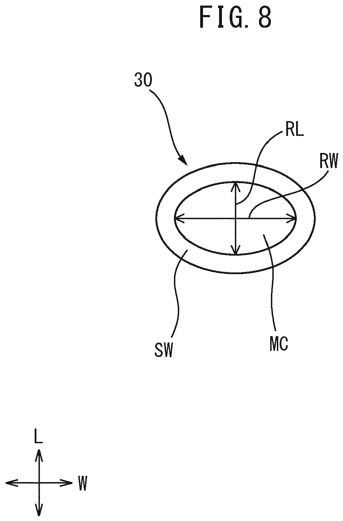

The flatness of the surface of the core portion MC can be evaluated by a line roughness test on the surface of the core portion MC (according to JISB0601-1944). Fig. 8 is a schematic view for describing a line roughness test of the fused portion. Fig. 8 schematically shows the position of the fused portion 30 where the line roughness test is performed in a plan view when evaluating the flatness of the surface of the core portion MC. In the line roughness test, the line roughness is measured along the line segment RL having the maximum dimension parallel to the longitudinal direction L, and the line roughness is measured along the line segment RW having the maximum dimension parallel to the lateral direction W. Specifically, the method of the line roughness test is as follows.

### <Line Roughness Test on Surface of Core Portion>

(1) A portion of the disposable diaper 1 including a part of the joining portion 14 (including the fused portion 30) is cut out into a size of 25 mm in the longitudinal direction L × 25 mm in the lateral direction W as a sample. (2) The sample is disposed in the measuring portion of KS1100 manufactured by KEYENCE Co., Ltd. (3) In the core portion MC of the fused portion 30, the line roughness (surface roughness) is measured along the line segment RL of the maximum dimension in the longitudinal direction L to obtain the arithmetic average roughness Ra, and the line roughness (surface roughness) is measured along the line segment RW of the maximum dimension in the lateral direction W to obtain the arithmetic average roughness Ra. (4) A product of the arithmetic average roughness (Ra (longitudinal direction L) × Ra (lateral direction W)) is obtained. The average value of the products of the arithmetic average roughness of at least five samples is calculated as the final value.

In the present embodiment, the fact that the surface of the core portion MC is "flat" means that the product of the arithmetic average roughness in the longitudinal direction L (Ra (longitudinal direction L)) and the arithmetic average roughness in the lateral direction W (Ra (lateral direction W)) is 0.15 µm² or less. In the case where the surface of the core portion MC is "flat", the thickness dT of the core portion MC is almost even, and the core portion MC is substantially homogeneous as a whole. Therefore, for the reasons described above, it can be said that the individual fused portions 30 are substantially homogeneously formed.

The index for evaluating the flatness of the surface of the core portion MC is not limited to the above example, and other indexes can be used. As other indexes, for example, each of the arithmetic average roughness in the longitudinal direction L (Ra (longitudinal direction L)) and the arithmetic average roughness in the lateral direction W (Ra (lateral direction W)) (for example, when both the arithmetic average roughness values are 0.5 µm or less, the surface is flat), the product of the root mean square roughness in the longitudinal direction L (RMS (longitudinal direction L)) and the root mean square roughness in the lateral direction W (RMS (lateral direction W)) (for example, when the product is 0.2 µm² or less, the surface is flat), and each of the root mean square roughness in the longitudinal direction L (RMS (longitudinal direction L)) and the root mean square roughness in the lateral direction W (RMS (lateral direction W)) (for example, when both the root mean square roughness values are 0.8 µm or less, the surface is flat).

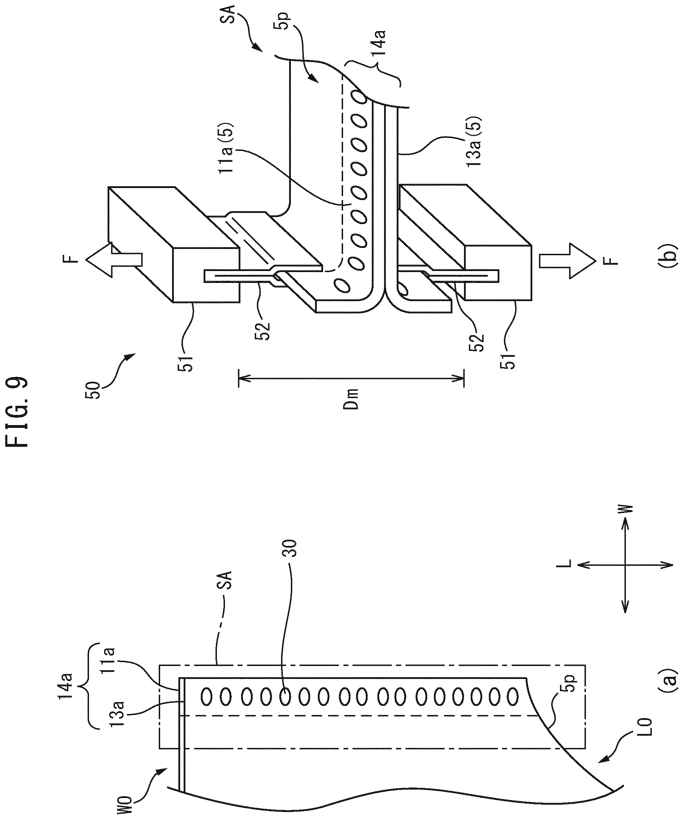

On the other hand, whether or not the plurality of fused portions are substantially homogeneous as a whole can be evaluated by a small variation in the peeling strength of the plurality of fused portions. This is because, since the peeling strength of the fused portion changes depending on the fusion state, when the peeling strengths are almost the same among the plurality of fused portions, that is, when the variation in the peeling strengths among the plurality of fused portions is small, the fusion states among the plurality of fused portions are almost the same. Whether or not the plurality of fused portions are substantially homogeneous as a whole can be evaluated by a peeling test between the cover sheet 5 of the stomach-side portion 11 and the cover sheet 5 of the back-side portion 13 in the joining portion 14a and/or the joining portion 14b (hereinafter, also referred to as "joining portion 14") having the plurality of fused portions 30. Fig. 9 is a schematic view for describing the peeling test of the joining portion. Fig. 9(a) shows a sample SA taken out from the disposable diaper, and Fig. 9(b) shows a testing machine 50 that performs a peeling test for the joining portion. In the peeling test of the joining portion, in the present embodiment, using the testing machine 50 for T-type peeling test, one end portion of the sample SA including the joining portion 14 in the longitudinal direction L is held between two chucks (jigs) 51 and 51 so as to widen a distance Dm between the two chucks 51 and 51, and while peeling off the joining portion 14a, the relationship between a load F applied between the two chucks 51 and 51 and the distance Dm is measured. Specifically, the peeling test of the joining portion is performed as follows.

### <Peeling Test of Joining Portion>

(1) The end portion of the disposable diaper 1 in the lateral direction W is cut out in a strip extending in the longitudinal direction L including the joining portion 14 from the end portion of the disposable diaper 1 on the waist opening WO side to the end portion on the leg opening LO side as a sample SA. The size of the sample SA is such that the length of the lateral direction W is approximately the sum of the length of the joining portion 14 and the width of the chuck, and the length of the longitudinal direction L is the length of the joining portion 14. In the present embodiment, the size of the sample SA is 60 mm in the lateral direction W× 200 mm in the longitudinal direction L. (2) Using the testing machine 50 for peeling test, the cover sheet 5 of the stomach-side portion 11 and the cover sheet 5 of the back-side portion 13 are respectively held with the two chucks 51 and 51 of the testing machine 50 from the longitudinal direction L side in a portion 5p that does not include the joining portion 14 in the lateral direction W at one end portion of the sample SA in the longitudinal direction L (for example, the end portion on the waist opening WO side) (the central part of the disposable diaper 1 before being cut out). At this time, in the present embodiment, the portion 5p of the cover sheet 5 is pinched between holding sheets 52 so that the cover sheet 5 can be easily held by the chucks 51, and the holding sheets 52 are held by the chucks 51. However, the distance (initial value) between the chucks 51 and 51 at the time of holding is set to 30 mm in advance. (3) In the testing machine 50, the two chucks 51 and 51 are pulled at a constant speed (for example, 500 mm/min) so as to widen the distance Dm between the two chucks 51 and 51, and while peeling off the cover sheet 5 of the stomach-side portion 11 and the cover sheet 5 of the back-side portion 13 in the joining portion 14 in the 180° direction, the distance Dm between the two chucks 51 and 51 and the load F applied to the two chucks 51 and 51 are measured. Then, the sample SA (joining portion 14) is peeled off from one end portion in the longitudinal direction L (for example, the end portion on the waist opening WO side) to the other end portion (for example, the end portion on the leg opening LO side). In the case where the sample SA is torn in the lateral direction W and cannot be peeled off during the peeling, the above (2) and (3) are performed again for the remaining portion of the sample SA that has not been peeled off. (4) The relationship (graph) between the load F applied between the two chucks 51 and 51 and the distance D between the two chucks 51 and 51 when the sample SA is completely peeled off from one end portion to the other end portion in the longitudinal direction L is obtained. This graph has a protruding portion in which the load F temporarily shows a local maximum value (peeling strength at the fused portion 30) at almost each position of the plurality of fused portions 30. From the graph, the local maximum value of each of a plurality of protruding portions corresponding to the plurality of fused portions 30, the average value of the plurality of the local maximum values in the plurality of protruding portions, and the variation of the plurality of the local maximum values with respect to the average value (for example, standard deviation) are calculated. However, the average value of at least three samples is calculated as the final evaluation value.

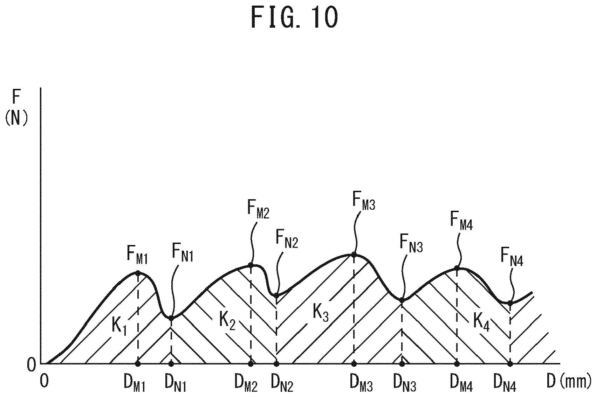

Fig. 10 is a graph showing an example of the relationship between the load F and the distance D in the peeling test of Fig. 9. The horizontal axis shows the distance D (=Dm-30 (initial value)) (mm) between the two chucks 51 and 51, that is, from one end edge of the sample SA in the longitudinal direction L which is the position where the peeling starts (for example, the end edge on the waist opening WO side). On the other hand, the vertical axis shows the load F (N) applied between the two chucks 51 and 51. As shown in the graph, the load F periodically alternately indicates a local maximum value F_{Mk} and a local minimum value F_{Nk}. However, k = 1 to n: n indicates the number of the fused portions 30. The position D_{Mk} showing the local maximum value F_{Mk} is substantially the position of the fused portion 30, and the position D_{Nk} indicating the local minimum value F_{Nk} is substantially the position between the fused portions 30 adjacent to each other. Therefore, the local maximum value F_{Mk} is the peeling strength at the fused portion 30 at the k-th position from the position, that is, the position where the peeling starts. The area of the region Kₖ interposed between the local minimum values F_{Nk-1} and F_{Nk} before and after the above position is the energy required for peeling at the k-th fused portion 30, and the total sum is the energy required for peeling off the entire joining portions 14. This graph can be seen as a graph having a plurality of protruding portions (regions Kₖ) corresponding to the plurality of fused portions 30. Therefore, from such a graph, it is possible to obtain the local maximum value F_{Mk} of each protruding portion (= region Kₖ), the average value of the local maximum values F_{Mk}, and the variation (for example, standard deviation) of the plurality of the local maximum values F_{Mk} with respect to the average value. In the actual measurement, the number of regions Kₖ is basically the same as the number of the plurality of fused portions 30 of the joining portion 14.

In the present embodiment, from the viewpoint that the plurality of fused portions 30 are substantially homogeneous as a whole, the standard deviation with respect to the average value of the local maximum values F_{Mk} in the plurality of protruding portions is 3 N or less in the graph. The upper limit of 3 N is set as follows. In the case where the wearer or caregiver is an elderly person, when the peeling strengths differ too much among the fused portions at the time of continuously pulling apart the joining portion, it is not possible to cope with the change such as a rapid increase or decrease in the peeling strength, and thus there is a concern that the plurality of fused portions may not be continuously peeled off. In the absorbent article, the upper limit of the change (standard deviation) in peeling strength is set to a value of 3 N based on the grip strength and grab strength of the elderly person to the extent that even such an elderly person can cope with.

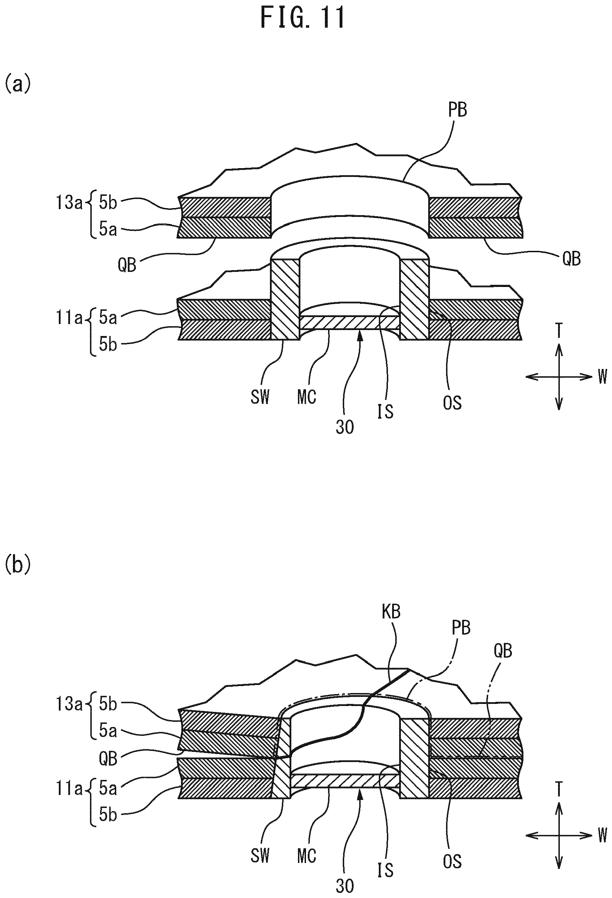

The action and effect of the disposable diaper 1 having the above-described constitution will be described with reference to Fig. 7 and Fig. 11. Fig. 11 is a schematic view for describing the appearance of the fused portion 30 being pulled apart. In the disposable diaper 1 of the present embodiment, each fused portion 30 in the joining portion 14a has the core portion MC and the peripheral wall portion SW. The peripheral wall portion SW is formed of the cover sheet 5 in the stomach-side portion 11 (stomach-side sheet member) and the cover sheet 5 in the back-side portion 13 (back-side sheet member) that are melted, integrated in the thickness direction, and solidified in a tubular wall shape. Therefore, usually, the peripheral wall portion SW is strong and is less likely to be broken compared with the cover sheet 5 in the stomach-side portion 11 or the back-side portion 13 in the periphery of the peripheral wall portion SW. Therefore, in the case of peeling off the joining portion 14a, the peripheral wall portion SW itself is less likely to be broken, and the cover sheet 5 in the stomach-side portion 11 or the back-side portion 13 in the periphery of the peripheral wall portion SW is easily broken at a boundary portion PB with the peripheral wall portion SW or the peripheral portion of the boundary portion PB (hereinafter, simply referred to as "boundary portion PB"). Therefore, as shown in Figs.7 (b) and 11(a), when the joining portion 14a is pulled apart to remove the disposable diaper 1, first, in a section other than the fused portion 30, it is possible to easily pull apart the joining portion 14a at a boundary QB between the cover sheet 5 of the stomach-side portion 11 and the cover sheet 5 in the back-side portion 13. In addition, in the fused portion 30 and the periphery of the fused portion 30, pulling is likely to occur at the boundary portion PB with (the peripheral wall portion SW of) the fused portion 30 in the cover sheet 5 of the stomach-side portion 11 or the back-side portion 13. Due to that, as shown in Fig. 11(a), for example, the stomach-side portion 11 and the back-side portion 13 of the joining portion 14a can be easily and continuously pulled apart from each other in the longitudinal direction L so as to form a hole in which the fused portion 30 remains on the cover sheet 5 of the stomach-side portion 11, and the fused portion 30 is removed from the cover sheet 5 of the back-side portion 13. In the present embodiment, such a form of peeling is also referred to as peeling of the fused portion 30, and the local maximum value F_{Mk} and energy associated with the peeling are also referred to as peeling strength and energy required for peeling, respectively.

However, in the case where the fusion of the fused portion 30 is incomplete, for example, when the width DP of the peripheral wall portion SW of the fused portion 30 is not even and partially small, the peripheral wall portion SW is partially weakened. Therefore, in the vicinity of such a fused portion 30, breakage or tearing easily occurs not at the boundary portion PB between the cover sheet 5 of the stomach-side portion 11 or the back-side portion 13 and the peripheral wall portion SW of the fused portion 30 but in the peripheral wall portion SW and the core portion MC. In such a case, for example, as shown in Fig. 11(b), during pulling apart the joining portion 14a, tearing KB occurring in the peripheral wall portion SW of the fused portion 30 may proceed to the cover sheet 5 in a section other than the joining portion 14a in the stomach-side portion 11 and the back-side portion 13, for example, the cover sheet 5 in the central part of the stomach-side portion 11 or the back-side portion 13. Due to that, there is a concern that it may be difficult to pull apart parts of the stomach-side portion 11 and the back-side portion 13 of the joining portion 14a continuously in the longitudinal direction L to the end, or it is necessary to repeat the pulling many times. In addition, for example, even when the tearing KB occurring in the peripheral wall portion SW of the fused portion 30 does not proceed to the cover sheet 5 in the central part of the stomach-side portion 11 and the back-side portion 13 and the like, in the case where a part of the plurality of fused portions 30 are such fused portions 30, pulling cannot be performed with almost the same force from the start to the end of the pulling and it is difficult to perform continuous pulling.

Therefore, the disposable diaper 1 of the present embodiment has the following constitution. First, the surface of the core portion MC is flat. That is, in the present embodiment, the product of the arithmetic average roughness (RaL) in the longitudinal direction L and the arithmetic average roughness (RaW) in the lateral direction W on the surface of the core portion MC is 0.15 µm² or less. Due to that, the thickness dT of the core portion MC is almost even, and the core portion MC is substantially homogeneous as a whole. Therefore, it can be said that the thickness DT and width DP of the peripheral wall portion SW formed at the same time as the core portion MC are also substantially even. That is, since the surface of the core portion MC is flat, it can be said that the fused portion 30 including the core portion MC is also substantially homogeneous. Therefore, each of the plurality of fused portions 30 is substantially homogeneous. From the viewpoint of further improving the flatness, the product of the arithmetic average roughness (RaL) in the longitudinal direction L and the arithmetic average roughness (RaW) in the lateral direction W is preferably 0.12 µm² or less and more preferably 0.10 µm² or less.

Further, in the peeling test of the joining portion, the standard deviation of the local maximum values F_{Mk} of the load F in the plurality of protruding portions (region Kk: k = 1 to n (n is the number of fused portions 30) corresponding to the plurality of fused portions 30) with respect to the average value is 3 N or less. Therefore, it can be said that the local maximum value F_{Mk} of the load F required for peeling each of the plurality of fused portions 30, that is, the peeling strength of each of the plurality of fused portions 30 is almost the same. Here, in general, the peeling strength of the fused portion changes depending on the state of fusion. Therefore, when the peeling strengths are almost the same among the plurality of fused portions 30, that is, when the change in the peeling strength among the plurality of fused portions 30 is small, it can be said that the fused states are almost the same among the plurality of fused portions 30. Therefore, when the plurality of fused portions 30 are viewed as a whole, it can be said that the fusion is properly performed and the plurality of fused portions 30 are substantially homogeneous as a whole. The standard deviation is preferably 2 N or less and more preferably 1.5 N or less.

As described above, in the present embodiment, since the individual fused portions 30 are substantially homogeneous and the plurality of fused portions 30 are substantially homogeneous as a whole, during the pulling of the joining portion 14a, the occurrence of tearing in a part of the fused portions 30 can be suppressed and the joining portion 14a can be continuously pulled apart with almost the same force from the start to the end of the pulling. That is, when the disposable diaper 1 having the joining portion 14a is removed, the stomach-side portion 11 and the back-side portion 13 can be appropriately separated continuously and stably with almost the same force without breaking the sheet members other than the joining portion 14a.

As a preferred embodiment of the present embodiment, in the peeling test of the joining portions 14 (14a, 14b), the average value of the local maximum values F_{Mk} in the plurality of protruding portions (regions Kₖ) corresponding to the plurality of fused portions 30 is 2 N or more and 12 N or less. In the disposable diaper 1, since the average value of the local maximum values F_{Mk} (peeling strength) is 2 N or more and 12 N or less, each fused portion 30 can be pulled apart from the cover sheet 5 of the stomach-side portion 11 or the back-side portion 13 with a relatively small force, and thus, the joining portion 14 can be pulled apart with a relatively small force. That is, the stomach-side portion 11 and the back-side portion 13 can be separated continuously and stably with almost the same force and easily with a relatively small force without breaking the cover sheet 5 in a section other than the joining portion 14. The average value of the local maximum values F_{Mk} is preferably 3 N or more and 11 N or less, and more preferably 4 N or more and 10 N or less. However, when the average value of the local maximum values F_{Mk} in the protruding portions (regions Kₖ) is less than 2 N, the peeling strength is too small, and thus the joining portion 14 may be peeled off while the disposable diaper 1 is being worn. On the other hand, when the average value of the local maximum values F_{Mk} in the protruding portions (regions Kₖ) is more than 12 N, the peeling strength is too large and thus it may be difficult to pull apart the joining portion 14 when undressing the disposable diaper 1. In particular, the upper limit of 12 N is set as follows. In the case where the wearer or caregiver is an elderly person, when the peeling strength of the fused portion is too large at the time of continuously pulling apart the joining portion, even if a small number of fused portions may be peeled off, there is a concern that all of the plurality of fused portions may not be continuously peeled off. In this absorbent article, the upper limit of the peeling strength is set to a value of 12 N based on the grip strength and grab strength of the elderly person to the extent that even such an elderly person can cope with.

As a preferred embodiment of the present embodiment, in the peeling test of the joining portion 14, the energy required for peeling off the joining portion 14 is 2 J or more and 7 J or less. In the disposable diaper 1, the energy required for peeling off the joining portion 14 is 2 J or more and 7 J or less, and thus the joining portion 14 can be pulled apart with a relatively small amount of work. That is, the stomach-side portion 11 and the back-side portion 13 can be separated continuously and stably with almost the same force and easily with a relatively small amount of work without breaking the cover sheet 5 in a section other than the joining portion 14. The energy is preferably 2 J or more and 6 J or less, and more preferably 2 J or more and 5 J or less. However, in the case where the energy required for peeling off the joining portion is less than 2 J, the amount of work is too small and the joining portion may be broken while the absorbent article is being worn. On the other hand, in the case where the energy required for peeling off the joining portion is more than 7 J, the amount of work is too large, and when undressing the absorbent article, there is a concern that it may be difficult to pull apart the joining portion. In particular, the upper limit of 7 J is set as follows. In the case where the wearer or caregiver is an elderly person, when the energy required for peeling is too high at the time of continuously pulling apart the joining portion, even if the joining portion may be peeled off halfway, there is a concern that the entire joining portion may not continuously peeled off. In this absorbent article, the upper limit of the energy required for peeling is set to a value of 7 J based on the grip strength and grab strength of the elderly person to the extent that even such an elderly person can peel off the entire joining portion.

As a preferred embodiment of the present embodiment, in the puncture test of the cover sheet 5 (stomach-side sheet member) of the stomach-side portion 11 and the cover sheet 5 (back-side sheet member) of the back-side portion 13, the maximum load of the cover sheet 5 of the stomach-side portion 11 and the maximum load of the cover sheet 5 of the back-side portion 13 are 10 N or more and 25 N or less. In the disposable diaper 1, since both the maximum loads (breaking strength) of the cover sheets 5 of the stomach-side portion 11 and the back-side portion 13 are 10 N or more and 25 N or less, the cover sheets 5 can be easily broken with a relatively small force when the stomach-side portion 11 and the back-side portion 13 are separated. Due to that, the cover sheet 5 is not broken in a section other than the joining portion 14, and the stomach-side portion 11 and the back-side portion 13 can be easily separated continuously and stably with almost the same force and easily with a relatively small force. The breaking strength is preferably 11 N or more and 24 N or less, and more preferably 12 N or more and 23 N or less. However, in the case where the maximum load of the cover sheet 5 is less than 10 N, the breaking strength is too small, and the joining portion 14 may be broken while the disposable diaper 1 is being worn. On the other hand, in the case where the maximum load of the cover sheet 5 is more than 25 N, the breaking strength is too large and it may be difficult to pull apart the joining portion 14. In particular, the upper limit of 25 N is set as follows. In the case where the wearer or caregiver is an elderly person, when the breaking strength of the cover sheet is too high at the time of continuously pulling apart the joining portion, even if the cover sheet can be broken in a small number of fused portions, there is a concern that the cover sheet may be continuously broken in all of the plurality of fused portions. In this absorbent article, the upper limit of the breaking strength is set to a value of 25 N based on the grip strength and grab strength of the elderly person to the extent that even such an elderly person can cope with.

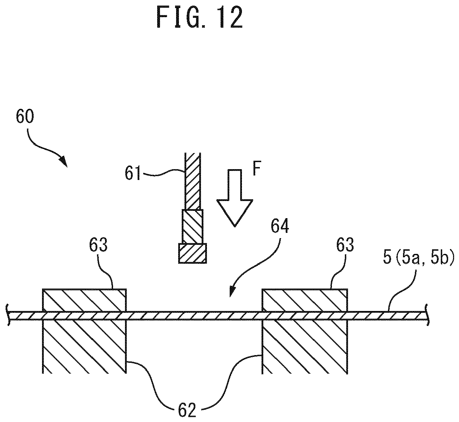

However, the puncture test of the cover sheets 5 of the stomach-side portion 11 and the back-side portion 13 is a test for measuring the breaking strength of the cover sheets 5 when a predetermined jig is punched into the cover sheets 5. Fig. 12 is a schematic view for describing a puncture test. In the puncture test, the cover sheet 5 is used as a sample, the sample is placed on the upper surface of a sample table 62 of a testing machine 60 and fixed with a fixture 63 or the like. Then, a jig 61 which is disposed to be inserted through a hole portion 64 which extends inward from the upper surface of the sample table 62 is lowered from above the sample and penetrated into the sample so as to be inserted through the hole portion 64. At that time, the load applied to the jig is measured. Specifically, the puncture test is performed as follows.

### <Puncture Test of Cover Sheet (Sheet Member)>

(1) The cover sheet 5 (unused) of the stomach-side portion 11 and the cover sheet 5 (unused) of the back-side portion 13 are cut out into a size of 100 mm × 100 mm, respectively, as samples. (2) Each sample (cover sheet 5) is placed on the upper surface of the sample table 62 and fixed with the fixture 63 or the like. The hole portion 64 (diameter 60 mm × depth 110 mm) extending toward the inside of the sample table 62 is formed on the upper surface of the sample table 62, and the sample is fixed so as to cover the hole portion 64. (3) The sample table 62 is set on a stand (not shown). The stand includes a columnar member (not shown) that extends vertically upward. A digital force gauge FGP-5 (manufactured by Nidec-Shimpo Co., Ltd.), in which the columnar jig 61 (diameter 11 mm × length 100 mm) is connected to the tip end of a gauge head, is attached to the columnar member. The jig 61 is disposed above the sample table 62 and is arranged so as to be inserted through the hole portion 64. (4) The jig 61 is lowered from above the sample at a speed of 500 mm/min, and the load applied to the jig 61 is measured until the sample is broken and the jig 61 penetrates the sample. The maximum load at this time is defined as the breaking strength (N) of the sheet member (cover sheet 5).

As a preferred embodiment of the present embodiment, in the puncture test, a difference between the maximum load of the cover sheet 5 (stomach-side sheet member) of the stomach-side portion 11 and the maximum load of the cover sheet 5 (back-side sheet member) of the back-side portion 13 is 1 N or more and 10 N or less. In the disposable diaper 1, since the difference in maximum load (breaking strength) between the cover sheet 5 of the stomach-side portion 11 and the cover sheet 5 of the back-side portion 13 is 1 N or more and 10 N or less, the cover sheet 5 having a relatively small maximum load can be relatively easily broken from each fused portion when the stomach-side portion 11 and the back-side portion 13 are separated. Due to that, the cover sheet 5 is not broken in a section other than the joining portion 14, and the stomach-side portion 11 and the back-side portion 13 are separated continuously and stably with almost the same force and more easily with a relatively small force. However, in the case where the difference in maximum load (breaking strength) between the cover sheet 5 of the stomach-side portion 11 and the cover sheet 5 of the back-side portion 13 is less than 1 N, the difference in breaking strength is too small and when the joining portion 14 is pulled apart, one of the two cover sheets 5 is broken in the vicinity of one fused portion 30 and the other is broken in the vicinity of another fused portion 30. The broken cover sheet 5 may be broken apart, making it difficult to continuously and stably separate the stomach-side portion 11 and the back-side portion 13. On the other hand, in the case where the difference in the maximum load (breaking strength) between the cover sheets 5 is more than 10 N, the difference in breaking strength is too large, and the cover sheet 5 having a weak breaking strength may be broken apart while the disposable diaper is being worn or it may be difficult to pull apart the cover sheet 5 having a strong breaking strength. In particular, the upper limit of 10 N is set as follows. In the case where the wearer or caregiver is an elderly person, when the difference in breaking strength between the cover sheets of the stomach-side portion and the back-side portion is large at the time of continuously pulling apart the joining portion, it is not possible to cope with the cover sheet having a strong breaking strength, and even if the cover sheet may be broken in a small number of fused portions, there is a concern that the cover sheet may not be continuously broken in all of the plurality of fused portions. In the absorbent article, the upper limit of the difference in breaking strength is set to a value of 10 N based on the grip strength and grab strength of the elderly person to the extent that even such an elderly person can cope with.

As a preferred embodiment of the present embodiment, a peeling test is performed with one end portion of the sample in the longitudinal direction L as each of the end portion of the waist opening WO side and the end portion of the leg opening LO side. In the disposable diaper 1, the peeling test is performed on each of the end portion of the waist opening WO side and the end portion of the leg opening LO side, and therefore, in each case, the standard deviation of the local maximum values F_{Mk} at least in the protruding portions (regions Kₖ) is 3N or less. Therefore, when the disposable diaper 1 having the joining portion 14 is removed, in any of the case where the joining portion 14 is pulled apart from the end portion on the waist opening WO side, and the case where the joining portion 14 is pulled apart from the end portion on the leg opening LO side, the stomach-side portion 11 and the back-side portion 13 can be appropriately separated continuously and stably with almost the same force and without breaking the cover sheet in a section excluding the joining portion 14. That is, the stomach-side portion 11 and the back-side portion 13 can be appropriately separated regardless of the pulling direction desired by the wearer or the caregiver.

Similarly, in the disposable diaper 1, a peeling test is performed on each of the end portion of the waist opening WO side and the end portion of the leg opening LO side, and in each case, it is preferable to satisfy the following requirements. That is, the average value of the local maximum value F_{Mk} in the plurality of protruding portions (regions Kₖ) corresponding to the plurality of fused portions 30 is preferably 2 N or more and 12 N or less, and the energy required for peeling the joining portion 14 is 2 J or more and 7 J or less.

In addition, as another embodiment, in the fused portion line 30L, the shapes of the plurality of fused portions 30 in the central region 22 and in the leg-side end region 23 may be different from the shapes of the plurality of fused portions 30 in the waist-side end region 21.

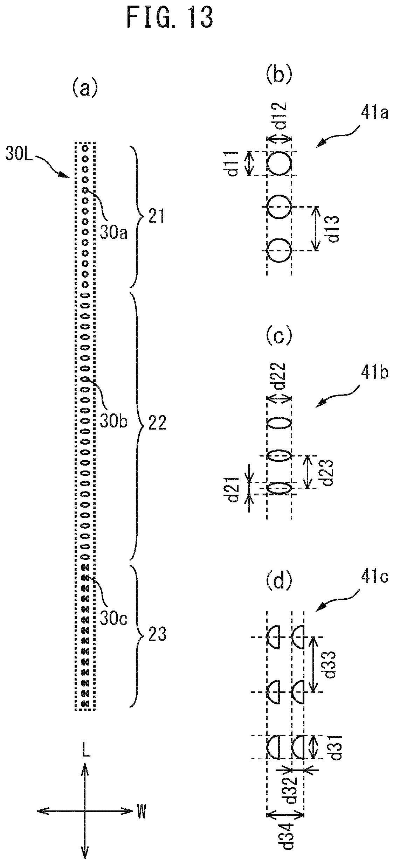

Fig. 13 is a plan view for describing another constitution example of the plurality of fused portions 30 in the joining portion 14a. Fig. 13(a) shows the fused portions 30a to 30c (fused portion line 30L) as seen from the side of the back-side portion 13 in the thickness direction T, and Figs. 13(b) to (d) show the shapes and dispositions of the protruding portions 41a to 41c of the anvil in which the fused portions 30a to 30c are formed by the ultrasonic wave sealing method. In the present embodiment, the respective fused portions 30a, 30b, and 30c in the waist-side end region 21, the central region 22, and the leg-side end region 23 have mutually different planar shapes. Therefore, in the case of forming the fused portions 30a, 30b, and 30c by the ultrasonic wave sealing method, the protruding portions 41a, 41b, and 41c of the anvil for the fused portions 30a, 30b, and 30c are different from each other. The planar shape of the protruding portion 41a of the anvil for forming the fused portion 30a in the waist-side end region 21 is a circular shape (truly circular shape), and a dimension d11 in the longitudinal direction L is equal to a dimension d12 in the lateral direction W. d11 and d12 are, for example, 0.5 mm to 5 mm. A distance d13 between the fused portions 30a adjacent to each other in the longitudinal direction L (the distance between the centers of the circles) is 1 mm to 30 mm. The planar shape of the protruding portion 41c of the anvil for forming the fused portion 30c in the leg-side end region 23 is a shape of a semicircle having a chord in the lateral direction W, and two protruding portions 41c are aligned close to each other in the lateral direction W. A dimension d31 in the longitudinal direction L, a dimension d32 in the lateral direction W, and a dimension d34 between both ends of the front end portion aligned in the lateral direction W are, for example, 0.5 mm to 5 mm. A distance d33 between the fused portions 30c adjacent to each other in the longitudinal direction L (the distance between the centers of the circles) is 1 mm to 30 mm.

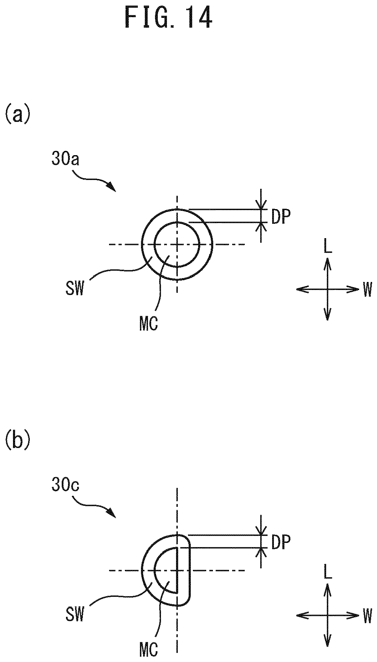

Fig. 14 is a plan view showing an example of the constitution of the fused portion 30 in the joining portion 14a. Fig. 14(a) shows the fused portion 30a, and Fig. 14(b) shows the fused portion 30c. The fused portion 30b is the same as the fused portion 30 in Fig. 7. Each of the fused portions 30a and 30c includes the core portion MC and the peripheral wall portion SW. The core portion MC and the peripheral wall portion SW are the same as the core portion MC and the peripheral wall portion SW of the fused portion 30 in Fig. 7 except that the shapes thereof in a plan view are different. In addition, it is not necessary that all of the fused portions in the joining portion 14a have the constitutions of the fused portion 30 or the fused portions 30a to 30c as described above. For example, only the fused portions in a portion that covers at least 30% of the joining portion 14a in the longitudinal direction L from the end edges 11ae and 13ae, which are portions on the side of the end portions 11e and 13e on the waist opening WO side where the joining portion 14a begins to be pulled apart (begins to break), may have the above-described constitution. Similarly, only the fused portions in a portion that covers at least 30% of the joining portion 14a in the longitudinal direction L from the end edges 11ae' and 13ae', which are portions on the side of the end portions 11e' and 13e' on the leg opening LO side where the joining portion 14a begins to be pulled apart (begins to break), may have the above-described constitution. In these cases, it is possible to easily start to pull apart the joining portion 14a.

### EXAMPLES

Hereinafter, the present invention will be more specifically described by exemplifying examples and comparative examples, however the present invention is not limited only to such examples.

### 1. Homogeneity of Joining Portion

### (1) Evaluation Target

Example 1: A disposable diaper shown in Figs. 1 to 7 was prepared. For the cover sheet on the stomach-side portion and the cover sheet on the back-side portion, a nonwoven fabric of 13 to 20 g/m² was used. Comparative Examples 1 to 3: Disposable diapers A, B, and C of other companies having almost the same structure as in Example 1 were used, respectively. Both the cover sheets on the stomach-side portion and the back-side portion were formed of a nonwoven fabric of 13 to 20 g/m².

### (2) Evaluation Method

### (2-1) Line Roughness Test for Surface of Core Portion

In the disposable diaper of Example 1, the line roughness test for the surface of the core portion was performed on the joining portion 14a on the left side in the lateral direction W as seen from the front side. Similarly, in the disposable diapers of Comparative Examples 1 to 3, the line roughness test for the surface of the core portion was performed on each of the joining portion on the left side and the joining portion on the right side in the lateral direction as seen from the front side.

### (2-2) Peeling Test of Joining Portion

In the disposable diaper of Example 1, the above peeling test of the joining portion was performed on each of the joining portion 14a on the left side and the joining portion 14b on the right side in the lateral direction W as seen from the front side. Similarly, in the disposable diapers of Comparative Examples 1 to 3, the peeling test of the joining portion was performed on each of the joining portion on the left side and the joining portion on the right side in the lateral direction as seen from the front side.

### (3) Results

### (3-1) Line Roughness Test for Surface of Core Portion

The evaluation results are shown in Table 1 below. In Table 1, Ra indicates the arithmetic average roughness, RMS indicates the root mean square roughness, W indicates that the line roughness is measured along the lateral direction W, and L indicates that the line roughness is measured along the longitudinal direction L. W × L indicates Ra (lateral direction W) × Ra (longitudinal direction L) and RMS (lateral direction W) × RMS (longitudinal direction L).

In the disposable diaper of Example 1, the product of the arithmetic average roughness (Ra) in the lateral direction W and the arithmetic average roughness (Ra) in the longitudinal direction L was 0.086 µm² and the value was 0.15 µm² or less. Therefore, the core portion of the disposable diaper of Example 1 was flat, and the individual fused portions 30 were substantially homogeneously formed. On the other hand, in the disposable diapers of Comparative Examples 1 to 3, the products of the arithmetic average roughness (Ra) in the lateral direction W and the arithmetic average roughness (Ra) in the longitudinal direction L were 0.214 µm², 0.171 µm², and 0.204 µm², and the values were more than 0.15 µm². Therefore, the core portions of the disposable diapers of Comparative Examples 1 to 3 were not flat, and the individual fused portions were not substantially homogeneously formed.

**[Table 1]**

| | Example 1 | | | Comparative Example 1 | | | Comparative Example 2 | | | Comparative Example 3 | | |
|---|---|---|---|---|---|---|---|---|---|---|---|---|
| Direction | W | L | W×L | W | L | W×L | W | L | W×L | W | L | W×L |
| Ra | 0.20 | 0.43 | 0.086 | 0.24 | 0.89 | 0.214 | 0.29 | 0.59 | 0.171 | 0.28 | 0.73 | 0.204 |
| RMS | 0.26 | 0.66 | 0.172 | 0.39 | 0.89 | 0.347 | 0.69 | 0.82 | 0.566 | 0.73 | 1.73 | 1.27 |

Regarding each of the arithmetic average roughness (Ra) in the lateral direction W and the arithmetic average roughness (Ra) in the longitudinal direction L, in Example 1, the arithmetic average roughness values were 0.20 µm and 0.43 µm, and both values are 0.5 µm or less. Thus, the core portion is flat. On the other hand, in Comparative Examples 1 to 3, the arithmetic average roughness value are 0.24 µm, 0.89 µm / 0.29 µm, 0.59 µm / 0.28 µm, and 0.73 µm, and the values are more than 0.5 µm. Thus, the core portion is not flat. In addition, regarding the product of the root mean square roughness (RMS) in the longitudinal direction L and the root mean square roughness (RMS) in the lateral direction W, in Example 1, the product is 0.172 µm² and the value is 0.2 µm² or less. Thus, the core portion is flat. On the other hand, in Comparative Examples 1 to 3, the products are 0.347 µm², 0.566 µm², and 1.27 µm² and the values are more than 0.2 µm². Thus, the core portion is not flat. Regarding each of the root mean square roughness in the longitudinal direction L and the root mean square roughness in the lateral direction W, in Example 1, the root mean square roughness values are 0.26 µm and 0.66 µm, and the both values are 0.8 µm or less. Thus, the core portion is flat. On the other hand, in Comparative Examples 1 to 3, the root mean square roughness values are 0.39 µm, 0.89 µm / 0.69 µm, 0.82 µm / 0.73 µm, and 1.73 µm, and the values are more than 0.8 µm. Thus, the core portion is not flat.

### (3-2) Peeling Test of Joining Portion

The evaluation results are shown in Tables 2 to 5 below. However, in Tables 2 to 5, W left indicates that the sample including the left side joining portion 14 of the disposable diaper is peeled from the end portion on the waist opening WO side. W right indicates that the sample including the right side joining portion 14 of the disposable diaper is peeled from the end portion on the waist opening WO side. L left indicates that the sample including the left side joining portion 14 of the disposable diaper is peeled off from the end portion on the leg opening LO side. L right indicates that the sample including the right side joining portion 14 of the disposable diaper is peeled off from the end portion on the leg opening LO side. E (J) indicates the energy required to peel off the joining portion 14 from one end portion on the waist opening WO side or the end portion on the leg opening LO side to the other end portion. The protrusion average (N) indicates the average value of the local maximum values F_{Mk} in the protruding portions (regions Kₖ). The lateral tearing (times) indicates the number of times in which the cover sheet is torn during peeling off the joining portion from one end portion on the waist opening WO side or the end portion on the leg opening LO side to the other end portion, from the joining portion 14 toward the central part of the disposable diaper (in the lateral direction W) and thus the peeling is interrupted. AVE indicates the average value of each value (E, protrusion average, and lateral tearing) in a plurality of samples, and STD indicates the standard deviation with respect to the average value.

In the disposable diaper of Example 1, as shown in Table 2, the standard deviation with respect to the average value of the local maximum values F_{Mk} in the protruding portions (regions Kₖ) in each of W left, W right, L left, and L right was 0.03 to 1.19, that is, 3 N or less. Since the standard deviation was 3 N or less, the peeling strength of each of the plurality of fused portions 30 was almost the same, and thus the plurality of fused portions 30 as a whole were substantially homogeneous. Therefore, in the disposable diaper of Example 1, the individual fused portions 30 were substantially homogeneously formed, and the average value of the number of times the peeling was interrupted, that is, the number of times the pulling of the joining portion was failed in the middle of the pulling was 0 to 0.33 times and was very small. As described above, since the individual fused portions 30 were substantially homogeneous and the plurality of fused portions 30 were substantially homogeneous as a whole, during the pulling of the joining portion, the occurrence of tearing in a part of the fused portions can be suppressed and the joining portion can be continuously pulled apart with almost the same force from the start to the end of the pulling. In addition, the average value of the local maximum values F_{Mk} in the protruding portions (regions Kₖ) was 7.45 N to 10.98 N, that is, 2 N or more and 12 N or less and was very small, and the joining portion could be pulled apart easily with a relatively small force. The average value of the energy E required for peeling off the joining portion was 2.32 J to 4.93 J, that is, 2 J or more and 7 J or less, and was very small, and the joining portion could be pulled apart easily with a relatively small amount of work.

In the disposable diapers of Comparative Examples 1 and 2, as shown in Tables 3 and 4, the standard deviation with respect to the average value of the local maximum values F_{Mk} in the protruding portions (regions Kₖ) was not included in a range of 3 N or less in a part or all of each of W left, W right, L left, and L right. The peeling strength of each of the plurality of fused portions was different, and therefore the plurality of fused portions as a whole were not substantially homogeneous. Therefore, in the disposable diapers of Comparative Examples 1 and 2, the individual fused portions were not substantially homogeneous, and the average value of the number of times the peeling was interrupted, that is, the number of times the pulling of the joining portion was failed in the middle of the pulling was 1.67 to 5.00 times and was very large. Further, in the disposable diaper of Comparative Example 3, as shown in Table 5, the standard deviation with respect to the average value of the local maximum values F_{Mk} in the protruding portions (regions Kₖ) in each of W left, W right, L left, and L right was 3 N or less. However, since the individual fused portions were not substantially homogeneous (Table 1), the average value of the number of times the peeling was interrupted, that is, the number of times the pulling of the joining portion was failed in the middle of the pulling, was 0.33 to 2.67 times and was very large. As described above, since the individual fused portions were not substantially homogeneous and the plurality of fused portions were not substantially homogeneous as a whole, a part of the fused portions were torn during the pulling of the joining portion, and it was not possible to continuously pull apart the joining portion with almost the same force from the start to the end of pulling. Further, the average value of the local maximum values F_{Mk} in the protruding portions (regions Kₖ) was larger than the range of 2 N to 12 N, a relatively large force was required to pull apart the joining portion, and the average value of the energy E required for peeling off the joining portion was larger than the range of 2 J to 7 J. A relatively large amount of energy was required to pull apart the joining portion.

**[Table 2]**

| | Example 1 | | | | | | | |
|---|---|---|---|---|---|---|---|---|
| | W left | | W right | | L left | | L right | |
| | AVE | STD | AVE | STD | AVE | STD | AVE | STD |
| E (J) | 2.78 | 0.02 | 2.32 | 0.23 | 4.93 | 0.37 | 4.17 | 0.26 |
| Protrusion average (N) | 8.15 | 0.03 | 7.45 | 0.55 | 10.98 | 1.19 | 9.17 | 0.16 |
| Lateral tearing (times) | 0.00 | 0.00 | 0.00 | 0.00 | 0.33 | 0.58 | 0.00 | 0.00 |

**[Table 3]**

| | Comparative Example 1 | | | | | | | |
|---|---|---|---|---|---|---|---|---|
| | W left | | W right | | L left | | L right | |
| | AVE | STD | AVE | STD | AVE | STD | AVE | STD |
| E (J) | 9.73 | 0.97 | 12.00 | 2.59 | 13.23 | 1.82 | 9.95 | 1.08 |
| Protrusion average (N) | 18.40 | 2.07 | 18.26 | 1.96 | 13.88 | 3.30 | 13.74 | 1.76 |
| Lateral tearing (times) | 1.67 | 0.58 | 3.33 | 1.15 | 4.33 | 0.58 | 2.67 | 0.58 |

**[Table 4]**

| | Comparative Example 2 | | | | | | | |
|---|---|---|---|---|---|---|---|---|
| | W left | | W right | | L left | | L right | |
| | AVE | STD | AVE | STD | AVE | STD | AVE | STD |
| E (J) | 8.33 | 1.08 | 8.18 | 1.23 | 9.43 | 0.81 | 8.67 | 1.36 |
| Protrusion average (N) | 18.92 | 7.14 | 16.35 | 3.71 | 10.32 | 1.02 | 9.39 | 1.02 |
| Lateral tearing (times) | 4.33 | 1.53 | 3.67 | 1.15 | 5.00 | 1.00 | 5.00 | 1.00 |

**[Table 5]**

| | Comparative Example 3 | | | | | | | |
|---|---|---|---|---|---|---|---|---|
| | W left | | W right | | L left | | L right | |
| | AVE | STD | AVE | STD | AVE | STD | AVE | STD |
| E (J) | 5.28 | 0.97 | 4.17 | 0.75 | 7.91 | 1.46 | 5.65 | 1.70 |
| Protrusion average (N) | 12.10 | 0.27 | 10.74 | 0.41 | 11.82 | 1.45 | 9.96 | 0.57 |
| Lateral tearing (times) | 0.33 | 0.58 | 0.33 | 0.58 | 2.67 | 1.53 | 1.67 | 1.16 |

### 2. Regarding Cover Sheet

### (1) Evaluation Target

Examples 2 to 5: As Example 2, the cover sheet on the back-side portion and the cover sheet on the stomach-side portion used for the disposable diaper of Example 1 were prepared. As Examples 3 to 5, the cover sheet on the back-side portion and the cover sheet on the stomach-side portion that could be used for the disposable diaper of Example 1 were prepared. As the cover sheets, a nonwoven fabric of 13 to 20 g/m² was used. Comparative Examples 4 to 6: The cover sheet of the back-side portion and the cover sheet of the stomach-side portion used for the disposable diapers A to C of other companies in Comparative Examples 1 to 3 were prepared. As the cover sheets, a nonwoven fabric of 15 to 18 g/m² was used.

### (2) Puncture Test of Cover Sheet

The above puncture test was performed on each of the cover sheets of Examples 2 to 5 and Comparative Examples 4 to 6.

### 3. Results

### (1) Maximum Load (Breaking Strength) of Cover Sheet

The evaluation results are shown in Table 1 below. However, in Table 6, O/B indicates the maximum load (breaking strength) of the cover sheet on the back-side portion, and O/T indicates the maximum load (breaking strength) of the cover sheet on the stomach-side portion.

In the cover sheets of Examples 2 to 5, the maximum load (breaking strength) of the cover sheet of the stomach-side portion and the maximum load (breaking strength) of the cover sheet of the back-side portion were 13.4 N to 21.6 N, and the values were 10 N or more and 25 N or less. Therefore, when the stomach-side portion and the back-side portion were separated, the cover sheet can be easily broken with a relatively small force. The difference between the maximum load of the cover sheet of the stomach-side portion and the maximum load of the cover sheet of the back-side portion was 1.6N to 8.2N, and the value was 1 N or more and 10 N or less. Therefore, when the stomach-side portion and the back-side portion are separated, the cover sheet having a relatively small maximum load can be relatively easily broken.

In the cover sheets of Comparative Examples 4 to 6, the difference between the maximum load of the cover sheet of the stomach-side portion and the maximum load of the cover sheet of the back-side portion was smaller than the range of 1 N to 10 N excluding the cover sheet of Comparative Example 6. Due to that, when the stomach-side portion and the back-side portion are separated, it is difficult to relatively easily break any one of the cover sheets, and it is difficult to continuously and stably separate the stomach-side portion and the back-side portion.

**[Table 6]**

| | O/B (N) | O/T (N) | Δ (N) |
|---|---|---|---|
| Example 2 | 13.4 | 21.6 | 8.2 |
| Example 3 | 13.4 | 15.0 | 1.6 |
| Example 4 | 19.4 | 21.6 | 2.2 |
| Example 5 | 19.4 | 15.0 | 4.4 |
| Comparative Example 4 | 17.4 | 17.1 | 0.3 |
| Comparative Example 5 | 21.3 | 21.0 | 0.3 |
| Comparative Example 6 | 21.4 | 12.5 | 8.9 |

### REFERENCE SIGNS LIST

1 Disposable diaper
11 Stomach-side portion
13 Back-side portion
11e, 13e End portion
14a Joining portion
30 Fused portion
MC Core portion
SW Peripheral wall portion

## Claims

1. An absorbent article having a longitudinal direction (L), a lateral direction (W), and a thickness direction (T) that are orthogonal to each other, and including a stomach-side portion (11) including a stomach-side sheet member, a back-side portion (13) including a back-side sheet member, and a joining portion (14a, 14b) in which at least one end portion (11a, 11b) of the stomach-side portion (11) and at least one end portion (13a, 13b) of the back-side portion (13) in the lateral direction (W) are joined to each other in a state of being overlapped in the thickness direction (T) along the longitudinal direction (L),
wherein the joining portion (14a, 14b) includes a plurality of fused portions (30) which are separated from each other and disposed along the longitudinal direction (L),
each of the plurality of fused portions (30) includes
a core portion (MC) in which the stomach-side sheet member and the back-side sheet member are fused to each other in the thickness direction (T), and
a peripheral wall portion (SW) in which the stomach-side sheet member and the back-side sheet member are fused to each other in the thickness direction (T) to extend in a tubular shape in the thickness direction (T) from a peripheral edge of the core portion (MC),
a surface of the core portion (MC) is flat,
in a peeling test of the joining portion (14a, 14b) in which one end portion of a sample (SA) including the joining portion (14a, 14b) in the longitudinal direction (L) is held by two jigs (51), and while peeling off the joining portion (14a, 14b) to widen a distance between the two jigs, a load (F) to be applied between the two jigs is measured, a graph showing the load (F) with respect to the distance (D) has a plurality of protruding portions (Kₖ) in which the load temporarily shows local maximum values (F_{Mk}) at respective positions of the plurality of fused portions (30), and
in the peeling test, when the one end portion of the sample (SA) in the longitudinal direction (L) is an end portion of either a waist opening (WO) side or a leg opening (LO) side of the absorbent article, a standard deviation with respect to an average value of the local maximum values (F_{Mk}) in the plurality of the protruding portions (Kₖ) corresponding to the plurality of fused portions (30) is 3 N or less, measured as described in the description.

2. The absorbent article according to claim 1, wherein in the peeling test, the average value of the local maximum values (F_{Mk}) in the plurality of protruding portions (Kₖ) corresponding to the plurality of fused portions (30) is 2 N or more and 12 N or less, measured as described in the description.

3. The absorbent article according to claim 1 or 2, wherein in the peeling test, an energy required for peeling off the joining portion (14a, 14b) is 2 J or more and 7 J or less, measured as described in the description.

4. The absorbent article according to any one of claims 1 to 3, wherein in a puncture test of the stomach-side sheet member and the back-side sheet member, a maximum load of the stomach-side sheet member and a maximum load of the back-side sheet member are 10 N or more and 25 N or less, measured as described in the description.

5. The absorbent article according to claim 4, wherein in the puncture test, a difference between the maximum load of the stomach-side sheet member and the maximum load of the back-side sheet member is 1 N or more and 10 N or less, measured as described in the description.

## Patentansprüche

1. Absorbierender Artikel, der eine Längsrichtung (L), eine seitliche Richtung (W) und eine Dickenrichtung (T) aufweist, die senkrecht zueinander sind, und der Folgendes einschließt: einen Bauchseitenteil (11), der ein Bauchseitenlagenelement einschließt, einen Rückenseitenteil (13), der ein Rückenseitenlagenelement einschließt, und einen Verbindungsteil (14a, 14b), in dem mindestens ein Endteil (11a, 11b) des Bauchseitenteils (11) und mindestens ein Endteil (13a, 13b) des Rückenseitenteils (13) in der seitlichen Richtung (W) in einem Zustand der Überlappung in der Dickenrichtung (T) entlang der Längsrichtung (L) miteinander verbunden sind,
wobei der Verbindungsteil (14a, 14b) eine Vielzahl von verschweißten Teilen (30) einschließt, die voneinander beabstandet und entlang der Längsrichtung (L) angeordnet sind,
wobei jeder der Vielzahl von verschweißten Teilen (30) Folgendes einschließt:
einen Kernteil (MC), in dem das Bauchseitenlagenelement und das Rückenseitenlagenelement in der Dickenrichtung (T) miteinander verschweißt sind, und
einen umlaufenden Wandteil (SW), in dem das Bauchseitenlagenelement und das Rückenseitenlagenelement in der Dickenrichtung (T) miteinander verschweißt sind, um sich in einer röhrenförmigen Form in der Dickenrichtung (T) von einem umlaufenden Rand des Kernteils (MC) zu erstrecken,
wobei eine Oberfläche des Kernteils (MC) flach ist,
wobei in einem Peel-Test des Verbindungsteils (14a, 14b), in dem ein Endteil einer Probe (SA), der den Verbindungsteil (14a, 14b) in der Längsrichtung (L) einschließt, von zwei Vorrichtungen (51) gehalten wird, und während des Abziehens des Verbindungsteils (14a, 14b), um einen Abstand zwischen den zwei Vorrichtungen zu vergrößern, eine Beanspruchung (F), die zwischen den zwei Vorrichtungen anzulegen ist, gemessen wird, wobei eine Kurve, die die Beanspruchung (F) in Bezug auf den Abstand (D) zeigt, eine Vielzahl von herausstehenden Teilen (Kₖ) aufweist, in denen die Beanspruchung temporär lokale maximale Werte (F_{Mk}) an jeweiligen Positionen der Vielzahl von verschweißten Teilen (30) zeigt, und
wobei in dem Peel-Test, wenn der eine Endteil der Probe (SA) in der Längsrichtung (L) ein Endteil von entweder einer Taillenöffnungs (WO)-Seite oder einer Beinöffnungs (LO)-Seite des absorbierenden Artikels ist, eine Standardabweichung in Bezug auf einen Durchschnittswert der lokalen maximalen Werte (F_{Mk}) in der Vielzahl der herausstehenden Teile (Kₖ), die der Vielzahl von verschweißten Teilen (30) entspricht, 3 N oder weniger beträgt, wie in der Beschreibung beschrieben gemessen wird.

2. Absorbierender Artikel nach Anspruch 1, wobei in dem Peel-Test der Durchschnittswert der lokalen maximalen Werte (F_{Mk}) in der Vielzahl von herausstehenden Teilen (Kₖ), die der Vielzahl von verschweißten Teilen (30) entspricht, 2 N oder mehr und 12 N oder weniger beträgt, wie in der Beschreibung beschrieben gemessen wird.

3. Absorbierender Artikel nach Anspruch 1 oder 2, wobei in dem Peel-Test eine Energie, die zum Abziehen des Verbindungsteils (14a, 14b) erforderlich ist, 2 J oder mehr und 7 J oder weniger beträgt, wie in der Beschreibung beschrieben gemessen wird.

4. Absorbierender Artikel nach einem der Ansprüche 1 bis 3, wobei in einem Durchstichtest des Bauchseitenlagenelements und des Rückenseitenlagenelements eine maximale Beanspruchung des Bauchseitenlagenelements und eine maximale Beanspruchung des Rückenseitenlagenelements 10 N oder mehr und 25 N oder weniger betragen, wie in der Beschreibung beschrieben gemessen wird.

5. Absorbierender Artikel nach Anspruch 4, wobei in dem Durchstichtest ein Unterschied zwischen der maximalen Beanspruchung des Bauchseitenlagenelements und der maximalen Beanspruchung des Rückenseitenlagenelements 1 N oder mehr und 10 N oder weniger beträgt, wie in der Beschreibung beschrieben gemessen wird.

## Revendications

1. Article absorbant ayant une direction longitudinale (L), une direction latérale (W) et une direction allant dans le sens de l'épaisseur (T) qui sont orthogonales les unes par rapport aux autres, et comprenant une partie côté ventre (11) comprenant un élément formant feuille côté ventre, une partie côté dos (13) comprenant un élément formant feuille côté dos, et une partie d'assemblage (14a, 14b) dans laquelle au moins une partie d'extrémité (11a, 11b) de la partie côté ventre (11) et au moins une partie d'extrémité (13a, 13b) de la partie côté dos (13) dans la direction latérale (W) sont assemblées l'une à l'autre dans un état de chevauchement dans la direction allant dans le sens de l'épaisseur (T) le long de la direction longitudinale (L),
dans lequel la partie d'assemblage (14a, 14b) comprend une pluralité de parties fusionnées (30) qui sont séparées les unes des autres et disposées le long de la direction longitudinale (L),
chaque partie de la pluralité de parties fusionnées (30) comprend
une partie centrale (MC) dans laquelle l'élément formant feuille côté ventre et l'élément formant feuille côté dos sont fusionnés l'un à l'autre dans la direction allant dans le sens de l'épaisseur (T), et
une partie de paroi périphérique (SW) dans laquelle l'élément formant feuille côté ventre et l'élément formant feuille côté dos sont fusionnés l'un à l'autre dans la direction allant dans le sens de l'épaisseur (T) pour s'étendre en une forme tubulaire dans la direction allant dans le sens de l'épaisseur (T) à partir d'un bord périphérique de la partie centrale (MC),
une surface de la partie centrale (MC) est plate,
dans un essai de pelage de la partie d'assemblage (14a, 14b), dans lequel une partie d'extrémité d'un échantillon (SA) comprenant la partie d'assemblage (14a, 14b) dans la direction longitudinale (L) est maintenue par deux gabarits (51), et lors du décollage de la partie d'assemblage (14a, 14b) pour élargir une distance entre les deux gabarits, une charge (F) destinée à être appliquée entre les deux gabarits est mesurée, un graphique représentant la charge (F) par rapport à la distance (D) a une pluralité de parties saillantes (Kₖ) dans lesquelles la charge montre temporairement des valeurs maximales locales (F_{Mk}) à des positions respectives de la pluralité de parties fusionnées (30), et
dans l'essai de pelage, lorsque ladite une partie d'extrémité de l'échantillon (SA) dans la direction longitudinale (L) est une partie d'extrémité soit d'un côté d'ouverture au niveau de la taille (WO) soit d'un côté d'ouverture au niveau de la jambe (LO) de l'article absorbant, un écart type par rapport à une valeur moyenne des valeurs maximales locales (F_{Mk}) dans la pluralité des parties saillantes (Kₖ) correspondant à la pluralité de parties fusionnées (30) est de 3 N ou moins, mesuré tel qu'il est décrit dans la description.

2. Article absorbant selon la revendication 1, dans lequel, dans l'essai de pelage, la valeur moyenne des valeurs maximales locales (F_{Mk}) dans la pluralité de parties saillantes (Kₖ) correspondant à la pluralité de parties fusionnées (30) est de 2 N ou plus et de 12 N ou moins, mesuré tel qu'il est décrit dans la description.

3. Article absorbant selon la revendication 1 ou la revendication 2, dans lequel, dans l'essai de pelage, une énergie requise pour peler la partie d'assemblage (14a, 14b) est de 2 J ou plus et de 7 J ou moins, mesurée tel qu'il est décrit dans la description.

4. Article absorbant selon l'une quelconque des revendications 1 à 3, dans lequel, dans un test de perforation de l'élément formant feuille côté ventre et de l'élément formant feuille côté dos, une charge maximale de l'élément formant feuille côté ventre et une charge maximale de l'élément formant feuille côté dos sont de 10 N ou plus et de 25 N ou moins, mesurées tel qu'il est décrit dans la description.

5. Article absorbant selon la revendication 4, dans lequel, dans l'essai de perforation, une différence entre la charge maximale de l'élément formant feuille côté ventre et la charge maximale de l'élément formant feuille côté dos est de 1 N ou plus et de 10 N ou moins, mesurée tel qu'il est décrit dans la description.
